(19) Europäisches Patentamt
European Patent Office
Office européen des brevets

(11) Publication number: 0 376 724 A2

# (12) EUROPEAN PATENT APPLICATION

(21) Application number: 89313649.9

(22) Date of filing: 28.12.89

(51) Int. Cl.5: C07D 501/24, C07D 417/12, A61K 31/545

(30) Priority: 29.12.88 JP 334000/88

(43) Date of publication of application: 04.07.90 Bulletin 90/27

(84) Designated Contracting States: AT BE CH DE ES FR GB GR IT LI LU NL SE

(71) Applicant: TANABE SEIYAKU CO., LTD.
2-10, Dosho-machi 3-chome
Chuo-ku Osaka(JP)

(72) Inventor: Wada, Masao
No.12-3, Minamicho 3-chome
Warabi-shi Saitama-ken(JP)
Inventor: Saito, Kunio
No. 100-1, Dotecho 2-chome
Omiya-shi Saitama-ken(JP)
Inventor: Takamura, Norio
No. 20-6, Shinshiraoka 2-chome
Shiraokamachi
Minamisaitama-gun Saitama-ken(JP)
Inventor: Matsushita, Tadahiro
No. 27-6, Koganehara 1-chome
Matsudo-shi Chiba-ken(JP)
Inventor: Yamaguchi, Totaro
No. 4-7, Ryoke 7-chome
Urawa-shi Saitama-ken(JP)

(74) Representative: Baverstock, Michael George Douglas et al
BOULT, WADE & TENNANT 27 Furnival Street
London, EC4A 1PQ(GB)

## (54) Cephalosporin compounds.

(57) Cephalosporin compounds of the formula:

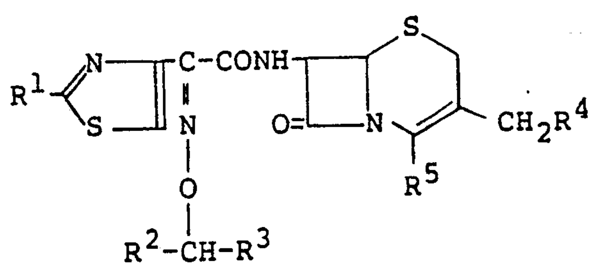

(I)

wherein $R^1$ is amino or protected amino; $R^2$ is substituted or unsubstituted heterocyclic group having 1 - 3 hetero atoms selected from oxygen and sulphur; $R^3$ is carboxyl or protected carboxyl; $R^4$ is nucleophilic compound residue; $R^5$ is carboxyl, protected carboxyl and $-COO^-$, a pharmaceutically acceptable salt thereof and their synthetic intermediate of the formula:

EP 0 376 724 A2

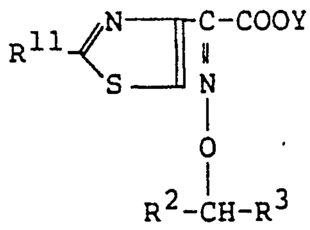

wherein $R^{11}$ is amino or protected amino; $R^2$ is substituted or unsubstituted heterocyclic group having 1 - 3 hetero atoms selected from oxygen and sulfur; $R^3$ is carboxyl or protected carboxyl; -COOY is carboxyl or protected carboxyl. Said cephalosporin compound is useful as antibacterial agent.

## CEPHALOSPORIN COMPOUNDS

This invention relates to a novel cephalosporin compound having a potent antimicrobial activity and synthetic intermediates thereof.

Hitherto, there have been known many cephalosporin antibiotics as anti-bacterial agents, for example, (6R, 7R)-7-[(Z)-2-(2-aminothiazol-4-yl)-2-((2-carboxyprop-2-yl)oxyimino)acetamido]-3-(1-pyridiniummethyl)-ceph-3-em-4-carboxylate (general name: Ceftazidime) has been disclosed to have a potent antimicrobial activity in Japanese Patent Second Publication (Kokoku) No. 5916/1987.

This invention relates to a novel cephalosporin compound of the following formula:

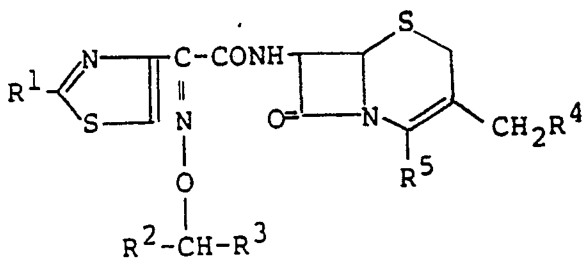

(I)

wherein $R^1$ is an amino group or a protected amino group; $R^2$ is a substituted or unsubstituted heterocyclic group having 1 - 3 hetero atoms selected from the group consisting of oxygen atom and sulfur atom; $R^3$ is a carboxyl group or a protected carboxyl group; $R^4$ is a nucleophilic residue; $R^5$ is a carboxyl group, a protected carboxyl group or a group of the formula: $-COO^-$, a pharmaceutically acceptable salt thereof and a synthetic intermediate thereof.

The novel cephalosporin compound of the present invention and a salt thereof show potent antimicrobial activity against a wide variety of microorganisms including gram-positive and gram-negative bacteria and are useful medicaments as anti-bacterial agents. For example, the desired compound (I) can be used in the treatment of infectious diseases caused by above gram-positive and gram-negative bacteria, as chemotherapeutic agents for warm-blooded animals, including human, and as nutritional supplements in animal food.

Preferred compounds of the present invention are the compounds of the formula (I) wherein a nucleophilic residue for $R^4$ is an acetoxy group, a pyridinio group having optionally a substituent, a quinolinio group having optionally a substituent, an isoquinolinio group, 2,3-cyclopentanopyridinio group, 5,6,7,8-tetrahydroquinolinio group, a phenantridinio group, a substituted thiazolinio group, a substituted tetrazolylthio group or a group of the formula:

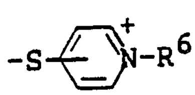

wherein $R^6$ is a lower alkenyl group, a lower alkyl group having optionally a substituent, a phenyl group having optionally a substituent or a nitrogen-containing heterocyclic group.

Among the above compounds, a therapeutically more preferred compounds are those of the formula (I) wherein a nucleophilic residue for $R^4$ is an acetoxy group; a pyridinio group; a pyridinio group having 1 - 3 substituents selected from the group consisting of an amino group, a lower alkyl group, a lower alkoxy group, a carbamoyl group and a phenyl group (e.g., 3-amino-2-methylpyridinio group, 5-amino-3-methoxypyridinio group, 3-carbamoylpyridinio group, 4-phenylpyridinio group); a quinolinio group, a quinolinio group substituted by a lower alkyl group, a lower alkoxy group or a halogen atom (e.g., 6-chloroquinolinio group, 6-fluoroquinolinio group, 6-bromoquinolinio group, 6-methylquinolinio group, 6-methoxyquinolinio group, 3-bromoquinolinio group, 4-chloroquinolinio group, 4-methylquinolinio group); an isoquinolinio group, 2,3-cyclopentanopyridinio group, 5,6,7,8-tetrahydroquinolinio group; a phenantridinio group; a lower alkyl group-substituted thiazolinio group (e.g., 4-methylthiazolinio group), a lower alkyl group-substituted tetrazolylthio group (e.g., 1-methyltetrazol-5-ylthio group); or a group of the formula:

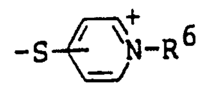

wherein $R^6$ is a lower alkenyl group (e.g., propenyl group); a lower alkyl group (e.g., methyl group); a lower alkyl group having a substituent selected from the group consisting of a lower alkylthio group, a phenyl group, a hydroxy group-substituted phenyl group ,a thiocarbamoyl group and an N-lower alkylthiocarbamoyl group (e.g., methylthiomethyl group, thiocarbamoylmethyl group, thiocarbamoylpropyl group, N-methylthiocarbamoylmethyl group, benzyl group, 3,4-dihydroxyphenethyl group); a phenyl group having a substituent selected from the group consisting of hydroxy group and amino group (e.g., 4-hydroxyphenyl group, 3,4-dihydroxyphenyl group, 3-amino-4-hydroxyphenyl group) or 2-oxo-pyrrolidinyl group.

Another examples of the compounds of the present invention are the compounds of the formula (I) wherein $R^1$ is an amino group; $R^3$ is a carboxyl group; $R^5$ is a carboxyl group or a group of the formula: $-COO^-$.

More preferred compounds of the present invention are compounds of the formula (I) wherein $R^2$ is a furyl group or a thienyl group.

The most preferred compounds are compounds of the formula (I) wherein $R^4$ is a quinolinio group, 6-chloroquinolinio group or a group of the formula:

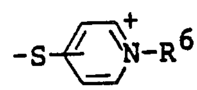

wherein $R^6$ is a thiocarbamoylmethyl group, 3,4-dihydroxyphenyl group or 3,4-dihydroxyphenethyl group.

Among the compounds (I) of the present invention, when $R^1$ is a protected amino group, a wide variety of protecting groups which have been usually employed to protect an amino group in the peptide synthesis can be used as the protecting group. Examples of such a protecting group are a lower alkanoyl group such as formyl group, acetyl group or pivaloyl group; a mono-, di- or trihalogeno-lower alkanoyl group such as chloroacetyl group, trifluoroacetyl group; a lower alkoxycarbonyl group such as methoxycarbonyl group, ethoxycarbonyl group, tert.-butoxycarbonyl group; a substituted or unsubstituted benzyloxycarbonyl group such as benzyloxycarbonyl group and p-methoxybenzyloxycarbonyl group; a substituted or unsubstituted phenyl-lower alkyl group such as benzyl group, p-methoxybenzyl group and 3,4-dimethoxybenzyl group; a di- or triphenyl-lower alkyl group such as benzhydryl group and trityl group. On the other hand, when $R^3$ and $R^5$ are a protected carboxyl group, it is preferable that the protecting group on the carboxyl group can be easily removed by conventional removing means such as hydrolysis, acid treatment or reduction. Suitable examples of such protecting groups are a lower alkyl group such as methyl group, ethyl group and tert.-butyl group, a substituted or unsubstituted phenyl-lower alkyl group such as benzyl group, p-methoxybenzyl group and p-nitrobenzyl group, benzhydryl groupl a tri-lower alkylsilyl group such as trimethylsilyl group, and the like.

The moiety of the formula:

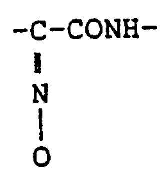

includes a geometrical isomer of the formula:

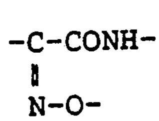

(Z)-isomer

or

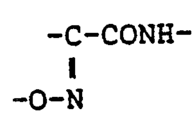

(E)-isomer

or a mixture thereof unless otherwise specified. However, when the desired compound (I) of the present invention is used as a medicament, the compound of the formula (I) wherein an oxyimino group has (Z)-configuration shows more excellent biological properties.

According to the present invention, the desired compound (I) or a pharmaceutically acceptable salt thereof can be prepared by the processes

(A) condensing an oxyiminoacetic acid compound of the formula:

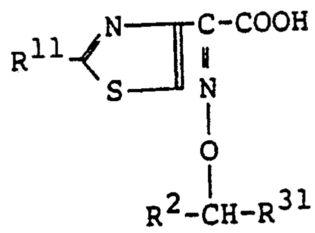

(II)

wherein $R^{11}$ is an amino group or a protected amino group; $R^{31}$ is a carboxyl group or a protected carboxyl group; $R^2$ is the same as defined above, a salt thereof or a reactive derivative thereof with a 7-aminocephalosporin compound of the formula:

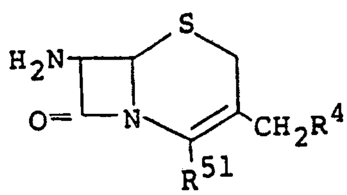

(III)

wherein $R^{51}$ is a carboxyl group, a protected carboxyl group or a group of the formula: $-COO^-$; $R^4$ is the same as defined above, or a salt, or

(B) reacting a cephalosporin compound of the formula:

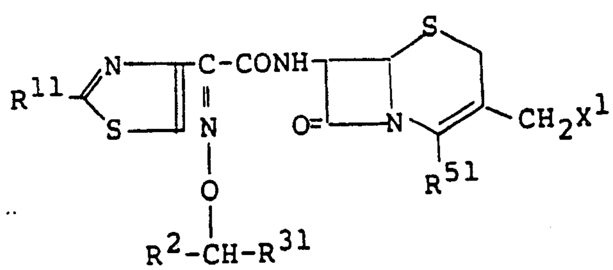

(IV)

wherein $X^1$ is a reactive group; $R^2$, $R^{11}$, $R^{31}$ and $R^{51}$ are the same as defined above, or a salt thereof with a nucleophilic compound (V) or a salt thereof, or

(C) condensing a cephalosporin compound of the formula:

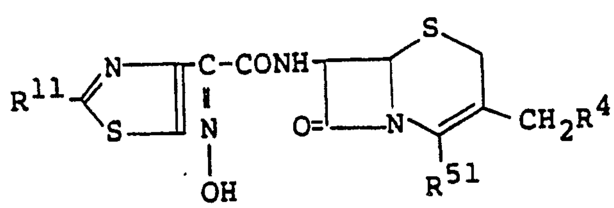

(VI)

wherein $R^{11}$, $R^4$ and $R^{51}$ are the same as defined above, or a salt thereof with a compound of the formula:

$$R^2\text{-}\underset{}{\overset{\overset{\displaystyle Z}{|}}{C}}\text{H-}R^{31} \qquad \text{(VII)}$$

wherein Z is a reactive group; $R^2$ and $R^{31}$ are the same as defined above, or a salt thereof, and

(D) when $R^{11}$ is a protected amino group and/or $R^{31}$ (and/or) $R^{51}$ are a protected carboxyl group, removing said protecting group therefrom, if desired, and

(E) converting the product into a pharmaceutically acceptable salt thereof, if desired.

The starting nucleophilic compound (V) includes acetic acid, pyridine, pyridine having a substituent (e.g., pyridine having 1 - 3 substituents selected from the group consisting of a lower alkyl group, a lower alkoxy group, amino group, carbamoyl group and phenyl group), quinoline, quinoline having a substituent (e.g., quinoline having a substituent selected from the group consisting of a lower alkyl group, a lower alkoxy group and a halogen atom), isoquinoline, 2,3-cyclopentanopyridine, 5,6,7,8-tetrahydroquinoline, phenanthridine, a lower alkyl group-substituted thiazole, (substituted tetrazoyl)thiol or a group of the formula:

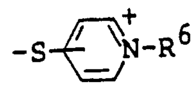

(wherein $R^6$ is the same as defined above), and these nucleophilic compounds (V) may be used in the form of a salt thereof (e.g., mineral acid salt, alkali metal salt) in the above reaction.

Conventional inorganic salts and organic amine salts can be used as a salt of the starting compounds (II), (III), (IV), (VI) and (VII) of the present invention.

In the compounds (II), (III), (IV), (VI) and (VII), when $R^{11}$ is a protected amino group and $R^{31}$and $R^{51}$ are a protected carboxyl group, these protecting groups may be the protecting groups for $R^1$, $R^3$ and $R^5$ as described above.

In the above process (A), the condensation reaction of a free oxyiminoacetic acid compound (II) with 7-aminocephalosporin compound (III) can be accomplished in the presence of a dehydrating agent in a suitable solvent. The dehydrating agent includes conventional dehydrating agents such as dicyclohexylcarbodiimide, phosphorus oxychloride, thionyl chloride, oxalyl chloride, and Vilsmeier reagent prepared from dimethylformamide and phosphorus oxychloride, oxalyl chloride, phosgene or thionyl chloride. Suitable examples of the solvent are dioxane, tetrahydrofuran, acetonitrile, methylene chloride, chloroform, pyridine, ethanol, water and a mixture thereof. It is preferred to carry out this reaction under cooling or at room temperature.

On the other hand, the condensation reaction of a reactive derivative (e.g., acid anhydride, mixed acid anhydride, active ester, etc.) of the oxyiminoacetic acid compound (II) with 7-aminocephalosporin compound (III) or a salt thereof can be conducted either in the presence or absence of a basic substance. This reaction can be accomplished in the same solvent as described above. Suitable examples of the basic substance are alkali metal hydroxides, alkali metal carbonates, alkali metal hydrogen carbonates, trialkylamines, N,N-dialkylanilines, pyridine and the like. It is preferred to carry out this reaction under cooling or at room temperature.

In the above process (B), suitable examples of the starting compound (IV) are the compounds wherein the reactive group for $X^1$ is a carbamoyl group, a lower alkanoyloxy group or a halogen atom and the like. It is preferred to carry out this reaction at room temperature or with heating in a solvent. Suitable examples of the reaction solvents are water, hydrophilic organic solvent such as dimethylformamide, dimethylacetamide, dioxane, acetone, alcohol, acetonitrile, dimethylsulfoxide, tetrahydrofuran, etc., or a mixture thereof. It is preferred to carry out this reaction at pH 2 - 8, especially at pH 5 -8. If necessary, in order to promote the reaction, these may be added alkali metal halides, alkali metal thiocyanates, trihalogenomethanesulfonic acid trialkylsilyl esters, alkali metal hydrogen carbonates, quaternary ammonium salts having surface active property (e.g., tri-lower alkylbenzylammonium halide), phosphate buffer solution, and the like.

In the above process (C), suitable examples of the starting compound (VII) are the compounds wherein the reactive group for Z is hydroxy group, a halogen atom, an alkylsulfonyl group or an arylsulfonyl group and the like. This condensation reaction can be accomplished in the presence of a dehydrating agent or a basic substance in a suitable solvent. As the dehydrating agent, the combination of a di-lower alkyl azodicarboxylate (e.g., diethyl azodicarboxylate, dimethyl azodicarboxylate, diisopropyl azodicarboxylate) and a phosphine compound (e.g., triphenylphosphine, diphenylmethylphosphine) can preferablly be used. The basic substance may be any of the basic substances described in the process (A). Suitable examples of the solvent are dimethylformamide, dimethylsulfoxide, dioxane, tetrahydrofuran, ethyl ether, benzene, toluene, chloroform, dichloromethylene, acetonitrile or a mixture thereof. It is preferred to carry our the reaction at -60° C to room temperature.

Among the desired compounds thus obtained, when $R^{11}$ is a protected amino group and/or $R^3$ (and/or) $R^{51}$ are a protected carboxyl group, the protecting groups thereof may be removed if necessary. The removal of these protecting groups can be conducted by the conventional removing means such as

hydrolysis, solvolysis, acid treatment and reduction or a combination thereof which are chosen according to the kind of the protecting groups.

The desired compound (I) of the present invention and a salt thereof show potent antimicrobial activity against a wide variety of microorganisms including those belonging to the genera Enterococcus (e.g., E. faecalis), Staphylococcus (e.g., S. aureus, S. epidermidis), Klebsiella (e.g., K. pneumoniae), Morganella (e.g., M. morganii), Citrobacter (e.g. C. freundii) and Pseudomonas (P. aeruginosa), and particularly characterized in their potent antimicrobial activity against both gram-positive and gram-negative bacteria.

For example, the antimicrobial activity of 7β-{(Z)-2-(2-aminothiazol-4-yl)-2-[(2-furyl)(carboxy)methyloxy imino]acetamido}-3-[(1-quinolinio)methyl]-3-cephem-4-carboxylate sodium salt of the present invention against Enterococcus faecalis CN-478, Morganella morganii 6501, Klebsiella pneumoniae 5038 and Citrobacter freundii GN-346 are more than 4 times stronger than the above mentioned Ceftazidime.

The antimicrobial activity of 7β-{(Z)-2-(2-aminothiazol-4-yl)2-[(2-thienyl)(carboxy)methyloxyimino]-acetomido}-3-[(1-thiocarbamoylmethyl-4-pyridino)thiomethyl]-3-cephem-4-carboxylate sodium salt, 7β-{(Z)-2-(2-aminothiazol-4-yl)-2-[(2-furyl)(carboxy)methyloxyimino]acetamido}-3-[(1-thiocarbamoylmethyl-4-pyridinio)thiomethyl]-3-cephem-4-carboxylate sodium salt and 7β-{(Z)-2-(2-aminothiazol-4-yl)-2-[(2-furyl)-(carboxy)methyloxyimino]acetamido}-3-[(6-chloro-1-quinolinio)methyl]-3-cephem-4-carboxylate sodium salt of the present invention against Staphylococcus aureus 209P JC-1 are more than 4 times stronger than Ceftazidime.

Further, the antimicrobial activity of 7β-{(Z)-2-(2-aminothiazol-4-yl)-2-[(2-furyl)(carboxy)methyloxyimino]-acetamido}-3-[[1-(3,4-dihydroxyphenyl)-4-pyridino]thiomethyl]-3-cephem-4-carboxylate sodium salt and 7β-{(Z)-2-(2-aminothiazol-4-yl)-2-[(2-furyl)(carboxy)methyloxyimino]acetamido}-3-[[1-(3,4-dihydroxyphenethyl)-4-pyridinio]thiomethyl]-3-cephem-4-carboxylate sodium salt of the present invention against Pseudomonas aeruginosa 35 R are more than 32 times stronger than Ceftazidime.

Moreover, the compound (I) and a salt thereof show potent antimicrobial activity against bacteria belonging to the genera Escherichia (e.g., E. coli), Enterobacter (e.g., E. aerogenes, E. cloacae), Serratia (S. marcescens), Proteus (P. rettgeri), Shigella, and Salmonella.

Further, the compound (I) and a salt thereof shows potent protective effects against microbial infections caused by various bacteria including Staphylococcus aureus and Pseudomonas aeruginosa, because of its high absorbability or long-lasting therapeutic effects in living tissues. Moreover, the compound (I) and a salt thereof is characterized in that they have a high stability, especially against β-lactamase-producing bacteria. In addition, the compound (I) shows low toxicity and therefore shows high safety as a medicament.

The cephalosporin compound (I) of the present invention can be used for medical use either in the free form or in the form of a pharmaceutically acceptable salt thereof. Suitable examples of pharmaceutically acceptable salts of the compound (I) are non-toxic metallic salts such as sodium, potassium, calcium, magnesium or aluminium salts; salts with non-toxic amines such as trialkyl-amines (e.g., triethylamine), pyridine, ethanolamine, triethanolamine, dicyclohexylamine; salts with inorganic acids such as hydrochloric acid, sulfuric acid or hydrobromic acid; salts with organic acids such as oxalic acid or tartaric acid: addition salts with amino acids such as glycine, lysine, arginine, aspartic acid or glutamic acid; and the like.

The salt of the compound (I) of the present invention may be a salt with resin, for example, consisting of polystyrene resin containing amino group, quaternary amino group or sulfonic acid group or of a resin containing carboxyl group such as polyacrylic acid resin. Further, it may be a complex with a metal such as iron or copper, or with ammonium salts such as ammonium chloride. Consequently, the desired compound (I) of the present invention and a salt thereof should be construed that it includes all inner salt, addition salt, complex, solvate and hydrate thereof.

The cephalosporin compound (I) of the present invention and a salt thereof can be administered either orally or parenterally (e.g., intravenously, intramuscularly, subcutaneously). The cephalosporin compound (I) of the present invention can be used properly as conventional pharmaceutical preparations such as tablets, granules, capsules, powder and injections.

The dose of the cephalosporin compound (I) of the present invention or a salt thereof may vary depending on the method of administration, the age, weight or conditions of patients and diseases to be treated. In general, the preferred daily dose of said compound (I) or a salt thereof may be about 2 - 200 mg/kg, especially about 5 - 40 mg/kg.

In the above present invention, there are optical isomers of the desired compound (I), the starting compounds (II), (IV) and (VII) due to the moiety of the formula:

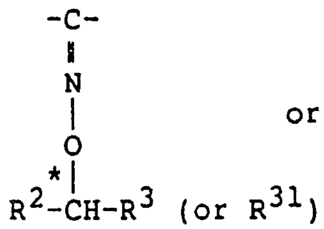

or

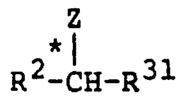

wherein * means an asymmetric carbon, $R^2$ and $R^3$ (or $R^{31}$) are the same defined above and Z is a reactive group, and the present invention includes these optical isomers and racemic compounds thereof.

The starting compound (II) of the present invention is a novel compound and can be prepared by reacting the compound (VII) with a compound of the formula:

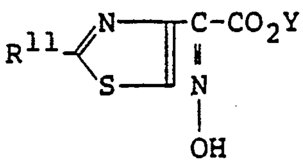

(VIII)

wherein $-CO_2Y$ is a carboxyl group or a protected carboxyl group, $R^{11}$ is the same as defined above, to give the compound of the formula:

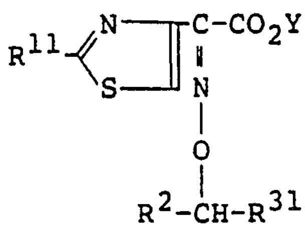

(IX)

wherein the all symbols are the same as defined above, or by reacting the compound (VII) with an alkali metal salt of N-hydroxyphthalimide to give the compound of the formula:

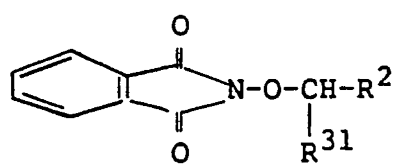

(X)

wherein the all symbols are the same as defined above, and then, further treating the said compound (X) with a lower alkylhydrazine to give the compound of the formula:

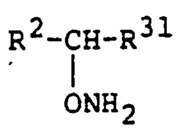

(XI)

wherein the all symbols are the same as defined above, and further, followed by reacting the said compound (XI) with a glyoxylic acid compound of the formula:

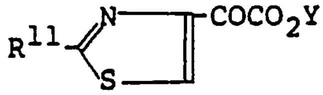

(XII)

wherein the all symbols are the same as defined above, and if necessary, by removing the protecting group from the said compound (XII).

The reaction of the compound (VII) with the compound (VIII) can be carried out in the same manner as that of the process (C) mentioned hereinbefore. It is preferred to carry out the reaction between the compound (VII) and an alkali metal salt of N-hydroxyphthalimide under cooling in a suitable solvent (e.g., dimethylformamide, dimethylsulfoxide, acetonitrile, tetrahydrofuran and a mixture thereof). It is preferred to carry out the treatment of the compound (X) with a lower alkylhydrazine (e.g., methylhydrazine) under cooling in a suitable solvent (e.g., methylene chloride, chloroform, acetonitrile, tetrahydrofuran and a mixture thereof). It is preferred to carry out the reaction between the compounds (XI) and (XII) in the presence or absence of a base for adjustment of pH value (e.g., alkali metal hydrogen carbonate, alkali metal carbonate, alkali metal hydroxide) under cooling - heating in a suitable solvent (e.g., acetonitrile, tetrahydrofuran, water, dioxane, dimethylformamide, dimethylsulfoxide and a mixture thereof). The removal of the protecting group from the thus obtained compound (IX) can be carried out in the same manners as the removal of the protecting group from the desired compound.

Throughout the specification and claims, the terms "lower alkyl" and "lower alkoxy" denote an alkyl and an alkoxy having 1 to 6 carbon atoms, especially 1 to 4 carbon atoms, respectively, and the terms "lower alkenyl" and "lower alkanoyl" denote an alkenyl and an alkanoyl having 2 to 6 carbon atoms, especially 2 to 4 carbon atoms, respectively.

The present invention is illustrated in detail by the following Examples and Reference Examples but should not be construed to be limited thereto.

Example 1

(1) To a suspension of 7-aminocephalosporanic acid (0.65 g) in acetonitrile (6 ml) is added 1,3-bis-(trimethylsilyl)urea (1.47 g) under ice cooling, and the mixture is stirred at room temperature for 4 hours (the resulting solution is referred to as "Solution A"). Separately, a suspension of 2-(2-tritylaminothiazol-4-yl)-2-[-(2-thienyl)(tert.-butyloxycarbonyl)methyloxyimino]acetic acid (1.25 g), 1-hydroxybenzotriazole (0.3 g) and dicyclohexylcarbodiimido (0.45 g) in dimethylformamide (6 ml) is stirred at room temperature for 1.5 hour (the resulting solution is referred to as "Suspension B"). Solution A is cooled with ice under argon atmosphere and thereto is added dropwise Suspension B, and then the mixture is stirred at room temperature for 22 hours. The mixture is poured into ice-water and extracted with ethyl acetate. The extract is washed with saline solution, dried, and then the solvent is distilled off under reduced pressure. The residue is dissolved in tetrahydrofuran (20 ml) and thereto is added diphenyldiazomethane (1.15 g). The mixture is stirred at room temperature for 2 hours. Further, the mixture is poured into ice-water and extracted with ethyl acetate. The extract is washed with aqueous sodium hydrogen carbonate solution and saline solution. After the mixture is dried, the solvent is distilled off under reduced pressure. The residue is purified by silica gel chromatography (solvent: hexane/ethyl acetate/chloroform) to give 7β-{(Z)-2-(2-tritylaminothiazol-4-yl)-2-[(2-thienyl)(tert.-butyloxycarbonyl)methyloxyimino]acetamido}cephalosporanic acid diphenylmethyl ester (0.85 g) as light brown powder.

$$IR\ \nu_{max}^{nujol} cm^{-1}: 3280, 1790, 1730, 1680$$

MS (m/z): 1046 ($MH^+$)

(2) Water (1 ml) is added dropwise into a solution of this product (0.82 g) in formic acid (4 ml), and the mixture is stirred at room temperature for 1.5 hour. The mixture is filtered and the filtrate is concentrated under reduced pressure to remove the solvent. Anisole (5 ml) is added to the residue and the mixture is cooled with ice. Trifluoroacetic acid (5 ml) is added thereto and stirred at room temperature for 4 hours. The mixture is concentrated under reduced pressure and the solvent is distilled off. The residue is powdered by treating with isopropyl ether and collected by filtration. The powder is dissolved in aqueous sodium hydrogen carbonate solution and purified by column chromatography on non-ionic adsorption polymer resin (trade name: Diaion HP-20; manufactured by Mitsubishi Chemical Industries, Ltd.; hereinafter referred to as HP-20) using water as a solvent. The fractions containing the desired product are collected, concentrated and then, lyophilized to give disodium 7β-{(Z)-2-(2-aminothiazol-4-yl)-2-[(2-thienyl) (carboxy)-methyloxyimino]acetamido}cephalosporanate (0.31 g) as colorless powder.

Yield: 64 %
M.p.: > 150° C (decomposed)

$$IR\ \nu_{max}^{nujol} cm^{-1}: 3340, 3200, 1760, 1600$$

MS (m/z): 626 ($MH^+$)
NMR ($D_2O$) δ : 2.11 (3H, s), 3.21 (1H, d, J = 18 Hz), 3.54 (1H, d, J = 18 Hz), 4.70 (1H, d, J = 12 Hz), 4.89 (1H, d, J = 12 Hz), 5.09 (1H, d, J = 4.8 Hz), 5.72 (1H, d, J = 4.8 Hz), 5.77 (1H, s), 7.04 (1H, s), 7.07 (1H, d, d, J = 5.4, 3.1 Hz), 7.25 (1H, d, J = 3.1 Hz), 7.48 (1H, dd, J = 5.4, 1 Hz)

Example 2

(1) Phosphorus oxychloride (0.73 g) and methylene chloride (3 ml) are added to dimethylformamide (0.57 g) successively under ice cooling under argon atmosphere, and the mixture is stirred at room temperature for 1 hour and then cooled to -60° C. To this mixture is added dropwise 2-(2-tritylaminothiazol-4-yl)-2-[(2-furyl)(diphenylmethyloxycarbonyl)methyloxyimino]acetic acid (2.47 g) in methylene chloride (25 ml) and stirred at -60° C to -55° C for 50 minutes (the resulting solution is referred to as "Reaction Solution A"). Separately, bis(trimethylsilyl)acetamide (3.85 ml) is added to a suspension of 7-aminocephalosporanic acid (1.42 g) in methylene chloride (15 ml) with ice cooling under argon atmosphere, and the mixture is stirred at room temperature for 40 minutes (the resulting solution is referred to as "Reaction Solution B"). Reaction Solution A is cooled to -60° C and thereto is added dropwise Reaction Solution B. The mixture is stirred at -60° C to -30° C for 2.5 hours. After the reaction, the mixture is poured into ice-water and extracted with ethyl acetate. The extract is washed with water, dried, and therefrom the solvent is distilled off to give 7β-{(Z)-2-(2-tritylaminothiazol-4-yl)-2-[(2-furyl)(diphenylmethyloxycarbonyl)methyloxyimino]-acetamido}cephalosporanic acid (3.37 g) as a crude product.

(2) This crude product (3.37 g) is dissolved in formic acid (45 ml) and thereto is added water (5 ml) under ice cooling, and the mixture is stirred at room temperature for 6 hours. After the reaction, water (5 ml) is added to the mixture and the insoluble materials are filtered off and the filtrate is concentrated to remove the solvent. Ethanol is added to the residue and the precipitates are collected by filtration, washed with ether and then dried to give 7β-{(Z)-2-(2-aminothiazol-4-yl)-2-[(2-furyl)(carboxy)methyloxyimino]-acetamido}cephalosporanic acid (1.58 g) as light yellow powder.
Yield: 84 %
M.p.: >150° C (decomposed)

$$IR\ \nu_{max}^{nujol} cm^{-1}: 3300, 3200, 2200\text{-}2800, 1770, 1730$$

MS (m/z): 566 ($MH^+$)
NMR (DMSO-$d_6$) δ : 2.03 (3H, s), 3.24 - 3.80 (4H, m), 4.67 and 4.69 (1H, dX2, J = 13.7 Hz and 12.7 Hz), 4.99 and 5.00 (1H, dX2, J = 12.7 Hz and 13.7 Hz), 5.07 and 5.12 (1H, dX2, J = 4.9 Hz and 4.9 Hz), 5.62 and 5.65 (1H, sX2), 5.75 and 5.79 (1H, dX2, J = 4.9 Hz and 4.9 Hz), 6.50 (1H, m), 6.58 (1H, m), 6.80 and 6.82 (1H, sX2), 7.71 (1H, s), 9.54 (1H, d, J = 7.8 Hz)
Disodium salt of this product:
M.p.: > 160° C (decomposed)

$$IR\ \nu_{max}^{nujol} cm^{-1}: 3350, 3200, 1770, 1610$$

MS (m/z): 610 ($MH^+$)

Example 3

(1) 2-(2-Tritylaminothiazol-4-yl)-2-[(S)-(2-furyl)(diphenylmethyloxycarbonyl)methyloxyimino]acetic acid

(641 mg) and 7-aminocephalosporanic acid (413 mg) are treated in the same manners as in Example 2-(1) to give 7β-{(Z)-2-(2-tritylaminothiazol-4-yl)-2-[(S)-(2-furyl)(diphenylmethyloxycarbonyl)methyloxyimino]-acetamido}cephalosporanic acid diphenylmethyl ester (727 mg).
Yield; 84 %
M.p.; 188 - 189 °C (decomposed), $[\alpha]_D^{20}$ + 19.7°(C = 1.0,chloroform)

(2) This product (708 mg) and 98 % formic acid (10 ml) are treated in the same manners as in Example 2-(2) to give 7β-{(Z)-2-(2-aminothiazol-4-yl)-2-[(S)-(2-furyl)(carboxy)methyloxyimino]-acetamido}cephalosporanic acid (454 mg) as light yellow powder.
M.p.: >150 °C (decomposed)

$$IR\ \nu_{max}^{nujol} cm^{-1}: 3300 - 2200, 1770, 1720, 1670, 1630, 1220, 1020, 750$$

MS (m/z): 566 ($MH^+$), 309, 154, 137
MNR (DMSO-$d_6$) δ : 2.04 (3H, s), 2.9 - 3.80 (2H + $H_2O$, m), 4.67 (1H, d, J = 12.7 Hz), 4.98 (1H, d, J = 12.7 Hz), 5.07 (1H, d, J = 5.4 Hz), 5.59 (1H, s), 5.77 (1H, dd, J = 4.9, 7.8 Hz), 6.49 (1H, dd, J = 2.0, 3.4 Hz), 6.5 - 6.6 (1H, m), 6.80 (1H, s), 7.1 - 7.3 (2H, m), 7.6 - 7.7 (1H, m), 9.5 - 9.6 (1H, m)

Example 4

(1) 2-(2-Tritylaminothiazol-4-yl)-2-[(R)-(2-furyl) (diphenylmethyloxycarbonyl)methyloxyimino]acetic acid (721 mg) and 7-aminocepharosporanic acid (463 mg) are treated in the same manners as in Example 2-(1) to give 7β -{(Z)-2-(2-tritylaminothiazol-4-yl)-2-[(R)-(2-furyl)(diphenylmethyloxycarbonyl)methyloxyimino]-acetamido}-cephalosporanic acid (912 mg).
Yield: 94 %
M.p.: 151 - 153 °C (decomposed)

(2) This product (900 mg) and 98 % formic acid (10 ml) are treated in the same manners as in Example 2-(2) to give 7β-{(Z)-2-(2-aminothiazol-4-yl)-2-[(R)-(2-furyl)(carboxy)methyloxyimino]-acetamido}cephalosporanic acid (570 mg) as light yellow powder.
M.p.: >150 °C (decomposed).

$$IR\ \nu_{max}^{nujol} cm^{-1}: 3400 - 2300, 1770, 1720, 1670, 1630, 1230, 1030, 750$$

MS (m/z): 566 ($MH^+$), 506, 442, 167, 154
NMR (DMSO-$d_6$) δ : 2.04 (3H, s), 3.0 - 3.60 (1H + $H_2O$, m), 3.6 (1H, d, J = 18.1 Hz), 4.69 (1H, d, J = 12.7 Hz), 5.00 (1H, d, J = 12.7 Hz), 5.12 (1H, d, J = 4.9 Hz), 5.63 (1H, s), 5.77 (1H, dd, J = 4.9, 7.8 Hz), 6.49 (1H, dd, J = 1.7, 3.2 Hz), 6.59 (1H, d, J = 3.4 Hz), 6.79 (1H, s), 7.1 - 7.3 (2H, m), 7.6 - 7.7 (1H, m), 9.57 (1H, d, J = 7.8 Hz), 13.0 -14.0 (2H, brm)

Example 5

2-(2-Tritylaminothiazol-4-yl)-2-{(3-thienyl)(diphenylmethyloxycarbonyl)methyloxyimino]acetic acid (4.12 g) and 7-aminocephalosporanic acid (2.29 g) are treated in the same manners as in Example 2-(1) to give 7β-{(Z)-2-(2-tritylaminothiazol-4-yl)-2-[(3-thienyl)(diphenylmethyloxycarbonyl)methyloxyimino]-acetamido}cephalosporanic acid (6.07 g) as a crude product.

This crude product is treated in the same manners as in Example 2-(2) and dissolved in water and aqueous sodium hydrogen carbonate solution, and further purified by HP-20 column chromatography (solvent: water and 5 % aqueous methanol solution). The fractions containing the desired product are collected and concentrated. The residue is lyophilized to give 7β-{(Z)-2-(2-aminothiazol-4-yl)-2-[(3-thienyl)-(carboxy)methyloxyimino]acetamido}cephalosporanic acid disodium salt (2.39 g), as light yellow powder.
Yield: 76 %

M.p.: >150° C (decomposed)

$$IR\ \nu_{max}^{nujol} cm^{-1}: 3315\ (br),\ 3195,\ 1760,\ 1600\ (br)$$

MS (m/z): 648 ($MNa^+$), 626 ($MH^+$)
NMR ($D_2O$) δ : 2.106 and 2.114 (3H, sX2), 3.23 (1H, d, J = 18 Hz), 3.54 and 3.55 (1H, dX2, J = 18 Hz and 18 Hz), 4.70 and 4.73 (1H, dX2, J = 12 Hz and 13 Hz), 4.87 and 4.88 (1H, dX2, J = 12 Hz and 13 Hz), 5.095 and 5.101 (1H, dX2, J = 4.4 Hz and 4.9 Hz), 5.64 (1H, brs), 5.73 and 5.76 (1H, dX2, J = 4.4 Hz and 4.9 Hz), 7.03 (1H, s), 7.22 (1H, dd, J = 4.9, 1.5 Hz), 7.45 (1H, dd, J = 4.9, 2.9 Hz), 7.54 (1H, m)

Example 6

2-(2-Tritylaminothiazol-4-yl)-2-[(3-furyl)(diphenylmethyloxycarbonyl)methyloxyimino]acetic acid (5.08 g) and 7-aminocephalosporanic acid (2.87 g) are treated in the same manners as in Example 2-(1) to give 7β-{(Z)-2-(2-tritylaminothiazol-4-yl)-2-[(3-furyl)(diphenylmethyloxycarbonyl)methyloxyimino]-acetamido}cephalosporanic acid (9.29 g) as a crude product.

After this product is treated as in the same manners as in Example 2-(2), it is purified by HP-20 column chromatography (solvent: water and 20 % aqueous methanol solution). The fractions containing the desired product are collected and concentrated. The residue is lyophilized to give 7β-{(Z)-2-(2-aminothiazol-4-yl)-2-[(3-furyl) (carboxy)methyloxyimino]acetamido}cephalosporanic acid disodium salt (2.73 g) as light yellow powder.
Yield: 63 %
M.p.: 189 - 190 ° C (decomposed)

$$IR\ \nu_{max}^{nujol} cm^{-1}: 3310,\ 1760,\ 1730,\ 1650,\ 1600$$

MS (m/z): 632 ($MNa^+$)
NMR ($D_2O$) δ : 2.10 (3H, s), 3.29 (1H, d, J = 18.1 Hz), 3.59 and 3.60 (1H, dX2, J = 18.1 Hz and J = 18.1 Hz), 4.71 (1H, d, J = 12.7 Hz), 4.89 (1H, d, J = 12.7 Hz), 5.13 and 5.15 (1H, dX2, J = 4.9 Hz and 4.9 Hz), 5.53 and 5.54 (1H, sX2), 5.76 and 5.80 (1H, dX2, J = 4.9 Hz and 4.9 Hz), 6.58 (1H, d, J = 1 Hz), 7.04 (1H, s), 7.51 (1H, t), 7.67 (1H, d)

Example 7

(1) A solution of (R)-2-furyl-2-hydroxyacetic acid diphenylmethyl ester (0.31 g), 7β-{(Z)-2-(2-tritylaminothiazol-4-yl)-2-hydroxyiminoacetamido}cephalosporanic acid diphenylmethyl ester (0.85 g) and triphenylphosphine (0.39 g) in tetrahydrofuran (15 ml) is cooled to -60° C under argon atmosphere, and diethylazodicarboxylate (0.24 ml) is added thereto dropwise and then the mixture is stirred for 30 minutes at the same temperature. The mixture is further stirred for 20 hours at room temperature, and concentrated under reduced pressure. The residue is purified by silica gel chromatography (solvent: ethyl acetate/hexane) to give 7β-{(Z)-2-(2-tritylaminothiazol-4-yl)-2-[(S)-(2-furyl)(diphenylmethyloxycarbonyl)-methyloxyimino]acetamido}cephalosporanic acid diphenylmethyl ester (0.3 g) as light yellow powder.
M.p.: 110 - 113° C (decomposed)

(2) This product (0.28 g) is treated in the same manners as in Example 2-(2) to give 7β-{(Z)-2-(2-aminothiazol-4-yl)-2-[(S)-(2-furyl)(carboxy)methyloxyimino]acetamido}cephalosporanic acid (0.13 g) as light yellow powder.

The physico-chemical properties of this product are identified with those of the product obtained in Example 3-(2).

Example 8

To a suspension of 7β-amino-3-(1-quinolinio)methyl-3-cephem-4-carboxylate (0.34 g) in water (2.2 ml) and ethanol (3.5 ml) is added dropwise triethylamine (1.8 ml) at -20° C to -15° C, and the mixture is stirred

at the same temperature for 10 minutes (the resulting solution is referred to as "Solution A"). Separately, phosphorus oxychloride (0.37 ml) is added dropwise to a solution of (Z)-2-(2-tritylaminothiazol-4-yl)-2-[(S)-(2-furyl)(diphenylmethyloxycarbonyl)methyloxyimino]acetic acid (0.72 g) in dimethylacetamide (1.5 ml) and methylene chloride (1.5 ml) at -25 °C to -20 °C. The mixture is stirred at -10 °C to -5 °C for 5 minutes and then at -40 °C for 30 minutes (the resulting solution is referred to as "Solution B"). Solution A is added to Solution B, and then the mixture is stirred at -35 °C to -25 °C for 30 minutes. The mixture is poured into ice-water and extracted with methylene chloride. The extract is washed with water, dried, and then the solvent is distilled off to give 7β-{(Z)-2-(tritylaminothiazol-4-yl)-2-[(S)-(2-furyl)(diphenylmethyloxycarbonyl)-methyloxyimino]acetamido}-3-{(quinolinio)methyl]-3-cephem-4-carboxylate (1.0 g) as a light yellow crude product. The product is dissolved in formic acid (12 ml), and water (3 ml) is added thereto. The mixture is stirred at room temperature for 14 hours and the insoluble materials are filetered off. The insoluble materials are washed with 80 % formic acid. The filtrate and washing water are combined together and concentrated under reduced pressure. An aqueous acetonitrile is added to the residue, and the mixture is adjusted to pH 8 with aqueous sodium hydrogen carbonate solution. Acetonitrile is distilled off and the aqueous layer is purified by HP-20 column chromatography (solvent: water/20 % aqueous methanol). The fractions containing the desired product are collected and concentrated. The residue is lyophilized to give 7β-{(Z)-2-(2-aminothiazol-4-yl)-2-[(S)-(2-furyl)(carboxy)methyloxyimino]acetamido}-3-(1-quinolinio)methyl-3-cephem-4-carboxylate sodium salt (265 mg) as light yellow powder.

M.p.: >170 °C (decomposed)

$$IR\ \nu_{max}^{nujol} cm^{-1}: 3400 - 3300, 1770, 1600, 720$$

MS (m/z): 679 ($MNa^+$), 657 ($MH^+$), 484, 309, 177, 139, 119

NMR ($D_2O$) δ : 2.88 (1H, d, J = 17.1 Hz), 3.37 (1H, d, J = 18.6 Hz), 5.12 (1H, d, J = 4.9 Hz), 5.62 (1H, s), 5.76 (1H, d, J = 4.4 Hz), 5.86 (1H, d, J = 15.1 Hz), 5.95 (1H, d, J = 16.1 Hz), 6.19 (1H, dd, J = 2.0, 3.4 Hz), 6.48 (1H, d, J = 3.4 Hz), 6.98 (1H, s), 7.29 (1H, d, J = 1.0 Hz), 7.9 - 8.1 (2H, m), 8.1 - 8.3 (1H, m), 8.41 (1H, d, J = 8.3 Hz), 8.51 (1H, d, J = 9.3 Hz), 9.19 (1H, d, J = 8.3 Hz), 9.27 (1H, d, J = 5.9 Hz)

Example 9

A suspension of 7β-amino-3-[(1-pyridinio)methyl]-3-cephem-4-carboxylate (0.96 g) in methylene chloride (5 ml) and acetonitrile (5 ml) is cooled to -10 °C and thereto is added dropwise bis(trimethylsilyl)-acetamide (3.26 ml), and the mixture is stirred at -10 to -5 °C for 2 hours (the resulting solution is referred to as "Reaction Solution A"). Separately, A solution of phosphorus oxychloride (0.34 g) in methylene chloride (2 ml) is added dropwise into dimethylformamide (0.26 g) with stirring under ice cooling and the mixture is stirred at room temperature for 50 minutes and then cooled to -60 °C. To this solution is added dropwise a solution of 2-(2-tritylaminothiazol-4-yl)-2-[(2-thienyl)(tert.-butyloxycarbonyl)methyloxyimino]acetic acid (1.25 g) in methylene chloride (8 ml) and stirred at - 60 °C for 50 minutes (the resulting solution is referred to as "Reaction Solution B"). Reaction Solution A is added to Reaction Solution B at -60 °C and stirred at -60 to -20 °C for 50 minutes and then, water is added thereto to finish the reaction and extracted with methylene chloride. The extract is washed with water and dried. The solvent is distilled off and the residue is powdered with ether and collected by filtration. This powder (1.0 g) is dissolved in formic acid (6 ml) and thereto is added water (1.5 ml) under ice cooling. The mixture is stirred at room temperature for 1.5 hour and filtered. The filtrate is concentrated under reduced pressure. Trifluoroacetic acid (5 ml) and anisole (5 ml) are added to the residue and the mixture is stirred at room temperature for 4 hours. Ether is added to the mixture and the resulting powder is collected by filtration. This powder is dissolved in water and aqueous sodium hydrogen carbonate and purified by HP-20 column chromatography (solvent: water). The fractions containing the desired product are collected and concentrated. The residue is lyophilized to give 7β-{(Z)-2-(2-aminothiazol-4-yl)-2-[(2-thienyl)(carboxy)methyloxyimino]acetamido}-3-[(1-pyridinio)methyl]-3-cephem-4-carboxylate sodium salt (0.3 g) as light yellow powder.

M.p.: >150 °C (decomposed)

$$IR\ \nu_{max}^{nujol} cm^{-1}: 3350, 1770, 1660, 1610$$

NMR ($D_2O$) δ : 2.92 (1H, d, J = 18 Hz), 3.52 (1H, d, J = 18 Hz), 5.16 (1H, d, J = 4.9 Hz), 5.26 (1H, d, J = 14 Hz), 5.56 (1H, d, J = 14 Hz), 5.76 (1H, m), 5.76 (1H, s), 6.91 (1H, dd), 6.99 (1H, s), 7.19 (1H, m), 7.33 (1H, dd), 8.09 (2H, t), 8.58 (2H, t, J = 7.8 Hz), 8.94 (2H, d, J = 5.9 Hz)

Example 10

(1) Bis(trimethylsilyl)acetamide (2.14 ml) is added dropwise to a suspension of 7β-amino-3-[(1-methyl-1H-tetrazol-5-yl)thiomethyl]-3-cephem-4-carboxylic acid (0.79 g) in methylene chloride (7 ml) under ice cooling and the mixture is stirred at room temperature for 1 hour (the resulting solution is referred to as "Reaction Solution A"). Separately, a solution of phosphorus oxychloride (0.34 g) in methylene chloride (1 ml) is added dropwise to dimethylformamide (0.26 g) with stirring under ice cooling and the mixture is stirred at room temperature for 50 minutes and cooled to -60 °C. To this solution is added dropwise a solution of 2-(2-tritylaminothiazol-4-yl)-2-[(2-thienyl)(tert.butyloxycarbony)methyloxyimino]acetic acid (1.25 g) in methylene chloride (9 ml) at -60 °C and stirred at the same temperature for 1 hour (the resulting solution is referred to as "Reaction Solution B"). Reaction Solution A is added dropwise to Reaction Solution B at -60 °C and stirred at -60 to -20 °C for 1.5 hour. To the mixture is added water and extracted with chloroform. The extract is washed with water, dried and therefrom the solvent is distilled off. The residue is dissolved in tetrahydrofuran (20 ml) and thereto is added diphenyldiazomethane (1.2 g) and further stirred at room temperature for 2 hours. The mixture is concentrated under reduced pressure to remove the solvent and the residue is purified by silica gel chromatography (solvent: hexane/ethyl acetate) to give 7β-{(Z)-2-(2-tritylaminothiazol-4-yl)-2-[(2-thienyl)(tert.-butyloxycarbonyl)methyloxyimino]acetamido}-3-[(1-methyl-1H-tetrazol-5-yl)thiomethyl]-3-cephem-4-carboxylic acid diphenylmethyl ester (0.96 g) as powder.

$$IR\ \nu_{max}^{nujol} cm^{-1}: 3280, 1790, 1730, 1680, 1620$$

MS (m/z): 1102 ($MH^+$)

(2) This product (0.88 g) is dissolved in formic acid (8 ml) and thereto is added dropwise water (2 ml) under ice cooling and stirred at room temperature for 2 hours. Insoluble materials are filtered off and the filtrate is concentrated under reduced pressure to remove the solvent. To the residue (0.76 g) are added anisole (5 ml) and trifluoroacetic acid (5 ml) under ice cooling and the mixture is stirred at room temperature for 4 hours. Further the solvent thereof is distilled off under reduced pressure. Hexane is added to the residue and the resulting powder is collected by filtration. This powder is dissolved in water and aqueous sodium hydrogen carbonate solution and purified by HP-20 column chromatography (solvent: water). The fractions containing the desired product are collected and concentrated. The residue is lyophilized to give 7β- {(Z)-2-(2-aminothiazol-4-yl)-2-[(2-thienyl)(carboxy)methyloxyimino]acetamido}-3-[(1-methyl-1H-tetrazol-5-yl)-thiomethyl]-3-cephem-4-carboxylic acid disodium salt (0.25 g) as light yellow powder.

M.p.: >180 °C (decomposed)

$$IR\ \nu_{max}^{nujol} cm^{-1}: 3360, 1760, 1660, 1600$$

MS (m/z): 682 ($MH^+$)

NMR ($D_2O$) δ : 3.25 (1H, d, J = 18 Hz), 3.62 and 3.64 (1H, dX2, J = 18 Hz and 18 Hz), 4.00 and 4.02 (1H, dX2, J = 13 Hz and 13 Hz), 4.02 and 4.03 (3H, sX2), 4.29 and 4.32 (1H, dX2, J = 13 Hz and 13 Hz), 5.05 and 5.06 (1H, dX2, J = 4.9 Hz and 4.9 Hz), 5.65 and 5.68 (1H, dX2, J = 4.9 Hz and 4.9 Hz), 5.78 (1H, s), 7.03 (2H, m), 7.24 (1H, d), 7.46 (1H, d)

Example 11

7β-{(Z)-2-(2-Aminothiazol-4-yl)-2-[(2-thienyl)(carboxy)methyloxyimino]acetamido}cephalosporanic acid disodium salt (0.94 g), 5-formylamino-3-methoxypyridine (0.69 g) and sodium iodide (2.25 g) are dissolved in water (20 ml) at 80 °C and stirred at the same temperature under argon atmosphere for 50 minutes. After cooling, the mixture is purified by HP-20 column chromatography (solvent: water and 15 % aqueous

methanol solution) and therefrom the solvent is distilled off under reduced pressure. The residue is lyophilized to give 7β-{(Z)-2-(2-aminothiazol-4- yl)-2-[(2-thienyl)(carboxy)methyloxyimino]acetamido}-3-[(5-formylamino-3-methoxy-1-pyridinio)methyl]-3-cephem-4-carboxylate sodium salt (0.51 g) as light yellow powder. A suspension of this product (0.51 g) in 5 % hydrochloric acid (6 ml) is stirred at 40°C for 50 minutes and cooled. The mixture is alkalified with aqueous sodium hydrogen carbonate solution and purified by HP-20 column chromatography (solvent: 10 % aqueous methanol solution). The fractions containing the desired product are collected and concentrated. The residue is lyophilized to give 7β-{(Z)-2-(2-aminothiazol-4-yl)-2-[(2-thienyl)(carboxy)methyloxyimino]acetamido}-3-[(5-amino-3-methoxy-1-pyridinio)-methyl]-3-cephem-4-carboxylate sodium salt (0.35 g) as light yellow powder.
M.p.: >150°C (decomposed)

$$IR\ \nu_{max}^{nujol} cm^{-1}: 3330, 3200, 1770, 1600$$

MS (m/z): 668 ($MH^+$)
NMR ($D_2O$) δ : 2.75 and 2.84 (1H, dX2, J = 18 Hz and 18 Hz), 3.42 and 3.47 (1H, dX2, J = 18 Hz and 18 Hz), 3.89 and 3.91 (3H, sX2), 4.92 and 4.94 (1H, dX2, J = 15 Hz and 15 Hz), 5.35 and 5.37 (1H, dX2, J = 15 Hz and 15 Hz), 5.68 and 5.70 (1H, dX2, J = 4.9 Hz and 4.9 Hz), 5.76 (1H, s), 6.88 (1H, m), 6.93 and 6.95 (1H, sX2), 7.18 (2H, m), 7.33 (1H, dd), 7.87 (2H, m)

Example 12

7β-{(Z)-2-(2-Aminothiazol-4-yl)-2-[(2-furyl)-(carboxy)methyloxyimino]acetamido}cephalosporanic acid disodium salt and 3-formylamino-2-methylpyridine are treated in the same manners as in Example 11 to give 7β-{(Z)-2-(2-aminothiazol-4-yl)-2-[(2-furyl)(carboxy)methyloxyimino]-acetamido}-3-[(3-amino-2-methyl-1-pyridinio)methyl]-3-cephem-4-carboxylate sodium salt.
M.p.: >200°C (decomposed)

$$IR\ \nu_{max}^{nujol} cm^{-1}: 3360, 3200\ (sh), 1770, 1610\ (br)$$

MS (m/z): 658 ($MNa^+$)
NMR ($D_2O$) δ : 2.55 (3H, s), 2.87 and 2.89 (1H, dX2, J = 18 Hz and 18 Hz), 3.27 and 3.29 (1H, dX2, J = 18 Hz and 18 Hz), 5.12 (1H, d, J = 4.9 Hz), 5.30 (1H, brd), 5.51 (1H, d, J = 16 Hz), 5.61 and 5.63 (1H, sX2), 5.74 and 5.77 (1H, dX2, J = 4.9 Hz and 4.9 Hz), 6.32 - 6.37 (1H, m), 6.50 and 6.53 (1H, dX2, J = 2.9 and 3.4 Hz), 7.02 (1H, s), 7.43 (1H, m), 7.55 - 7.61 (1H, m), 7.71 (1H, d, J = 7.3 Hz), 8.07 (1H, d, J = 5.9 Hz)

Example 13

(1) Under argon atmosphere, trimethylsilyltrifluoromethane sulfonate (1.4 ml) is added to a solution of quinoline (1.1 g) in methylene chloride (10 ml) and the solution is stirred at room temperature for 10 minutes. Thereto is added 7β-{(Z)-2-(tritylaminothiazol-4-yl)-2-[(S)- (2-furyl)(diphenylmethyloxycarbonyl)-methyloxyimino]acetamido}cephalosporanic acid (974 mg) and the mixture is stirred at room temperature for 7 hours. The solvent is distilled off and diethyl ether and water are added to the residue. The mixture is stirred and the upper layer is removed. Water is added to the residue, and the precipitates are collected by the filtration, washed and then dried under reduced pressure to give 7β-{(Z)-2-(tritylaminothiazol-4-yl)-2-[-(S)-(2-furyl)(diphenylmethyloxycarbonyl)methyloxyimino]acetamido}-3-[(1-quinolinio)methyl]-3-cephem-4-carboxylate (630 mg) as light yellow powder.
M.p.: 143 - 145°C (decomposed)

$$IR\ \nu_{max}^{nujol} cm^{-1}: 3370, 3200, 1780, 1740, 1680$$

NMR (DMSO-$d_6$) δ : 3.10, 3.30 (2H, ABq, J = 18 Hz), 5.08 (1H, d, J = 4.9 Hz), 5.74 (1H, dd, J = 7.3, 4.9 Hz), 5.86 (1H, s), 6.04 (2H, brs), 6.32 (1H, m), 6.46 (1H, d, J = 3.4 Hz), 6.75 (1H, s), 6.89 (1H, s), 7.21 - 7.40 (25H, m), 7.54 (1H, brs), 8.09 (1H, m), 8.23 -8.35 (2H, m), 8.49 - 8.58 (2H, m), 9.01 (1H, brs), 9.32 -

9.42 (2H, m), 9.62 (1H, d, J = 7.3 Hz)

(2) To a solution of the product (1.04 g) in formic acid (12 ml) is added water (3 ml) and the mixture is stirred at room temperature for 12 hours. The insoluble materials are filtered off and washed with 80 % aqueous formic acid. The filtrate and washing solution are combined and concentrated under reduced pressure, and aqueous acetonitrile is added to the residue. The mixture is adjustsed to pH 8 with aqueous sodium hydrogen carbonate solution, and then acetonitrile is distilled off. The aqueous solution is purified by HP-20 column chromatography (solvent: water/20 % aqueous methanol). The fractions containing the desired product are collected and concentrated. The residue is lyophilized to give 7β-{(Z)-2-(2-aminothiazol-4-yl)-2-[(S)-(2-furyl)(carboxy)methyloxyimino]acetamido}-3-[(1-quinolinio)methyl]-3-cephem-4-carboxylate sodium salt (530 mg) as light yellow powder.

The physico-chemical properties of this product are identified with those of the product obtained in Example 8.

Example 14

7β-{(Z)-2-(2-Aminothiazol-4-yl)-2-[(2-furyl)(carboxy)methyloxyimino]acetamido}cephalosporanic acid disodium salt (0.85 g), quinoline (0.90 g) and sodium iodide (2.10 g) are dissolved in a mixture of water (15 ml) and acetonitrile (5 ml) and the pH value thereof is adjusted to pH 6.5 by adding acetic acid dropwise. Under argon atmosphere, the mixture is stirred at 80°C for 1 hour, and the solvent is distilled off. The pH value of the mixture is adjusted to pH 8.2 with aqueous sodium hydrogen carbonate solution and purified by HP-20 column chromatography (solvent: water/20 % aqueous methanol solution). The fractions containing the desired product are collected and concentrated and then lyophilized to give 7β-{(Z)-2-(2-aminothiazol-4-yl)-2-[(2-furyl)(carboxy)methyloxyimino]acetamido}-3-[(1-quinolinio)methyl]-3-cephem-4-carboxylate sodium salt (0.19 g), as light yellow powder.

M.p.: >200°C (decomposed)

$$IR\ \nu_{max}^{nujol} cm^{-1}: 3360, 1770, 1610$$

MS (m/z): 679 ($MNa^+$), 657 ($MH^+$)

NMR ($D_2O$) δ: 2.83 and 2.86 (1H, dX2, J = 18 Hz and 18 Hz), 3.30 and 3.35 (1H, dX2, J = 18 Hz and 18 Hz), 5.09 and 5.10 (1H, dX2, J = 4.9 Hz and 4.9 Hz), 5.56 and 5.61 (1H, sX2), 5.73 and 5.74 (1H, dX2, J = 4.9 Hz and 4.9 Hz), 5.87 (1H, d, J = 15 Hz), 5.92 (1H, d, J = 15 Hz), 6.13 and 6.19 (1H, ddX2, J = 3.4, 2.0 Hz and 3.4, 2.0 Hz), 6.38 and 6.48 (1H, dX2, J = 3.4 Hz and 3.4 Hz), 6.94 (1H, s), 7.29 (1H, d, J = 2.0 Hz), 7.99 -8.51 (5H, m), 9.17 (1H, d, J = 8.3 Hz), 9.25 (1H, d, J = 5.9 Hz)

Example 15

Under argon atmosphere, trimethylsilyltrifluoromethane sulfonate (1.4 ml) is added to a solution of 6-chloroquinoline (1.45 g) in methylene chloride (10 ml) and the mixture is stirred at room temperature for 10 minutes. Thereto is added 7β -{(Z)-2-(2-aminothiazol-4-yl)-2-[(2- furyl)(carboxy)methyloxyimino]-acetamido}cephalosporanic acid (0.59 g) and the mixture is stirred under refluxing for 4 hours. The precipitate is separated by filtration and the filtrate is concentrated . To the residue are added water (5 ml) and acetonitrile (10 ml) and the mixture is allowed to stand at room temperature for 20 minutes. The pH value of the mixture is adjusted to pH 8 by adding aqueous sodium hydrogen carbonate solution thereto and acetonitrile is distilled off. The aqueous solution is purified by HP-20 column chromatography (solvent: water and 5 - 30 % methanol). The fractions containing the desired product are collected and concentrated. The residue is lyophilized to give 7β-{(Z)-2-(2-aminothiazol-4-yl)-2-[(2-furyl)(carboxy)methyloxyimino]-acetamido}-3-[(6-chloro-1-quinolinio)methyl]-3-cephem-4-carboxylate sodium salt (300 mg) as light yellow powder.

M.p.: 206 - 210°C (decomposed)

$$IR\ \nu_{max}^{nujol} cm^{-1}: 3350 (br), 3200 (sh), 1770 (sh), 1610 (br)$$

MS (m/z): 550 ($MNa^+$-6-chloroquinoline), 528 ($MH^+$-6-chloroquinoline)
NMR ($D_2O$) δ: 2.87 and 2.88 (1H, dX2, J = 18 Hz and 18 Hz), 3.34 and 3.40 (1H, dX2, J = 18 Hz and 18 Hz), 5.11 and 5.14 (1H, dX2, J = 4.9 Hz and 4.9 Hz), 5.58 and 5.63 (1H, sX2), 5.74 and 5.75 (1H, dX2, J = 4.9 Hz and 4.9 Hz), 5.88 (2H, brs), 6.21 and 6.26 (1H, mX2), 6.44 and 6.51 (1H, dX2, J = 3.4 Hz and 3.4 Hz), 6.91 and 6.92 (1H, sX2), 7.36 (1H, m), 8.12 - 8.23 (2H, m), 8.40 (1H, d, J = 2.0 Hz), 8.50 and 8.52 (1H, dX2, J = 9.3 Hz and 9.3 Hz), 9.09 (1H, d, J = 8.8 Hz), 9.26 (1H, d, J = 5.4 Hz)

Examples 16 - 38

7β-{(Z)-2-(2-Aminothiazol-4-yl)-2-[(2-furyl or thienyl)(carboxy)methyloxyimino]-acetamido}cephalosporanic acid and nitrogen-containing heterocyclic compounds are treated in the same manners as in Examples 14 to 15 to give the compounds of the following Table 1.

Table 1

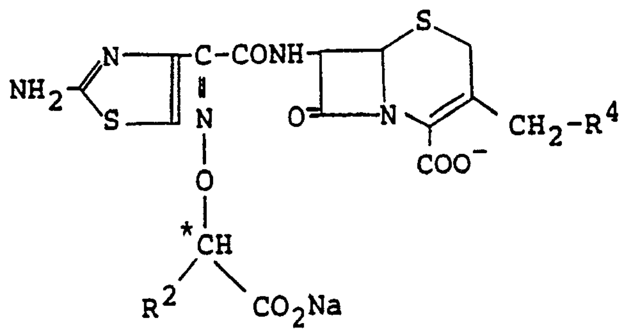

(I-A)

wherein the compound (I-A) is having Z-configuration; the symbol * means an asymmetric carbon atom; (S) and (R) in Table mean the configuration of asymmetric carbon atom thereof.

| Ex. No. | $R^2$ and $R^4$ | Compound (I-A)<br>Physicochemical properties |
|---|---|---|
| 16 | $R^2$:<br>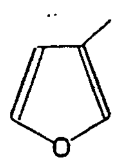<br>$R^4$:<br>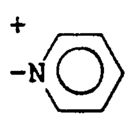 | M.p.: >200°C (decomposed)<br>IR $\nu_{max}^{nujol}$ $cm^{-1}$: 3330, 3190, 1770, 1650, 1610<br>MS (m/z): 607 ($MH^+$)<br>NMR ($D_2O$) δ : 3.00 and 3.13 (1H, dX2, J= 18 Hz and 18 Hz), 3.55 and 3.57 (1H, dX2, J = 18 Hz and 18 Hz), 5.19 and 5.21 (1H, dX2, J = 4.9 Hz and 4.9 Hz), 5.26 and 5.28 (1H, dX2, J = 14 Hz and 14 Hz), 5.57 and 5.59 (1H, dX2, J = 14 Hz and 14 Hz), 5.78 and 5.82 (1H, dX2, J = 4.9 Hz and 4.9 Hz), 6.49 and 6.53 (1H, dX2), 7.00 and 7.01 (1H, sX2), 7.33 and 7.40 (1H, tX2), 7.58 and 7.63 (1H, dX2), 8.11 (2H, m), 8.59 (1H, m), 8.96 (2H, dd) |
| 17 | $R^2$:<br>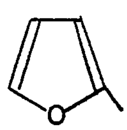<br>$R^4$:<br>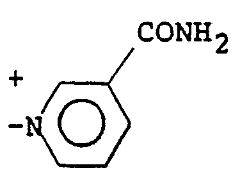 | M.p.: >180°C (decomposed)<br>IR $\nu_{max}^{nujol}$ $cm^{-1}$: 3340, 1770, 1610<br>MS (m/z): 650 ($MH^+$)<br>NMR ($D_2O$) δ : 2.98 and 2.99 (1H, dX2, J= 17 Hz and 17 Hz), 3.59 and 3,60 (1H, dX2, J = 17 Hz and 17 Hz), 5.19 (1H, d, J = 4.9 Hz), 5.35 (1H, d, J = 15 Hz), 5.62 - 5.80 (3H, m), 6.25 - 6.55 (2H, m), 7.01 (1H, s), 7.39 (1H, br), 8.25 (1H, m), 8.95 (2H, d), 9.16 (1H, d), 9.41 (1H, brs) |

| | | |
|---|---|---|
| 18 | $R^2$: 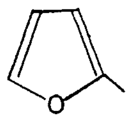 | M.p.: >200°C (decomposed) IR $\nu_{max}^{nujol}$ $cm^{-1}$: 3370, 3200, 1770, 1635 1610 MS (m/z): 705 ($MNa^+$), 683 ($NH^+$) NMR ($D_2O$) δ : 2.91 and 2.95 (1H, dX2, J = 18 Hz and 18 Hz), 3.52 and 3.56 (1H, dX2, J = 18 Hz and 18 Hz), 5.05 - 5.23 (2H, m), 5.49 - 5.62 (2H, m), 5.71 (1H, brd), 6.17 and 6.25 (1H, brX2), 6.40 and 6.48 (1H, dX2, J = 2.9 Hz and 2.9 Hz), 6.90 and 6.94 (1H, sX2), 7.32 and 7.36 (1H, brsX2), 7.62 (3H, m), 7.85 (2H, m), 8.27 (2H, m), 8.90 (2H, d, J = 5.9 Hz) |
| | $R^4$: 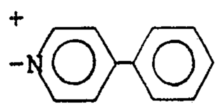 | |
| 19 | $R^2$: 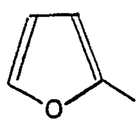 | M.p.: >200°C (decomposed) IR $\nu_{max}^{nujol}$ $cm^{-1}$: 3350, 3190, 1770, 1610 MS (m/z): 679 ($MNa^+$), 657 ($MH^+$) NMR ($D_2O$) δ : 2.88 and 2.96 (1H, dX2, J= 18 Hz and 18 Hz), 3.53 and 3,57 (1H, dX2, J = 18 Hz and 18 Hz), 5.17 and 5.18 (1H, dX2, J = 4.9 Hz and 4.9 Hz), 5.37 (1H, d, J = 14 Hz), 5.54 and 5.58 (1H, sX2), 5.70 (1H, d, J = 14 Hz), 5.73 (1H, d, J = 4.9 Hz), 5.94 and 6.07 (1H, ddX2, J = 3.4, 2.0 Hz and 3.4, 2.0 Hz), 6.28 and 6.43 (1H, dX2, J = 3.4 Hz and 3.4 Hz), 6.85 and 6.90 (1H, sX2), 7.20 and 7.23 (1H, dX2, J = 2.0 Hz and 2.0 Hz), 8.02 (1H, m), 8.22 (2H, m), 8.41 (1H, d, J = 6.8 Hz), 8.43 (1H, d, J = 6.8 Hz), 8.63 (1H, d, J = 6.4), 9.78 (1H, s), |
| | $R^4$: 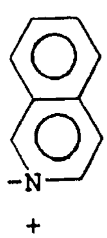 | |

| | | |
|---|---|---|
| 20 | $R^2$:<br>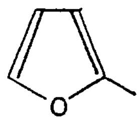<br>$R^4$:<br>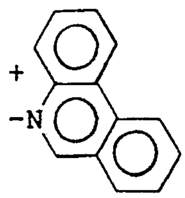 | M.p.: >200°C (decomposed)<br>IR $\nu_{max}^{nujol}$ $cm^{-1}$: 3350, 3200, 1770, 1610<br>MS (m/z): 729 ($MNa^+$)<br>NMR ($D_2O$) $\delta$ : 2.76 and 2.80 (1H, dX2, J= 18 Hz and 18 Hz), 3.21 and 3.30 (1H, dX2, J = 18 Hz and 18 Hz), 5.06 (1H, brs), 5.42 and 5.58 (1H, sX2), 5.69 (1H, d, J = 4.4 Hz), 5.86 (2H, brs), 5.92 and 6.05 (1H, brX2), 6.25 and 6.41 (1H, dX2, J = 2.9 Hz and 2.9 Hz), 6.83 (1H, s), 7.11 and 7.17 (1H, brsX2), 7.99 (3H, br), 8.26 - 8.41 (3H, m), 8.78 (2H, br), 9.74 (1H, brs) |
| 21 | $R^2$:<br>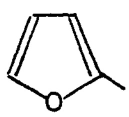<br>$R^4$:<br>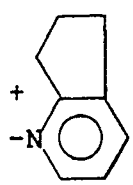 | M.p.: >200°C (decomposed)<br>IR $\nu_{max}^{nujol}$ $cm^{-1}$: 3360, 3200, 1770, 1610<br>MS (m/z): 669 ($MNa^+$), 647 ($MH^+$)<br>NMR ($D_2O$) $\delta$ : 2.31 (2H, m), 2.97 and 2.99 (1H, dX2, J = 19 Hz and 19 Hz), 3.15 - 3.43 (5H, m), 5.13 and 5.14 (1H, dX2, J = 4.9 Hz and 4.4 Hz), 5.31 (1H, d, J = 15 Hz), 5.40 (1H, d, J = 15 Hz), 5.61 and 5.63 (1H, sX2), 5.78 (1H, m), 6.27 - 6.38 (1H, m), 6.49 - 6.54 (1H, m), 7.005 and 7.012 (1H, sX2), 7.43 (1H, m), 7.78 (1H, m), 8.29 (1H, d, J = 7.8 Hz), 8.51 (1H, d, J = 5.9 Hz) |

| | | |
|---|---|---|
| 22 | $R^2$: 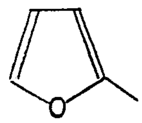 $R^4$: 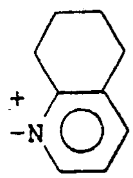 | M.p.: >200°C (decomposed)<br>IR $\nu_{max}^{nujol}$ $cm^{-1}$: 3360, 3200, 1770, 1610 (br)<br>MS (m/z): 683 ($MNa^+$), 661 ($NH^+$)<br>NMR ($D_2O$) δ : 1.70 - 2.00 (4H, m), 2.87 - 3.10 (5H, m), 3.00 and 3.35 (1H, dX2, J = 18 Hz and 18 Hz), 5.13 and 5.15 (1H, dX2, J = 4.4 Hz and 4.9 Hz), 5.24 (1H, d, J = 15 Hz), 5.46 (1H, d, J = 15 Hz), 5.61 and 5.64 (1H, sX2), 5.77 and 5.78 (1H, dX2, J = 4.9 Hz and 4.4 Hz), 6.30 - 6.39 (1H, m), 6.51 and 6.54 (1H, dX2, J = 3.4 Hz and 2.9 Hz), 6.99 and 7.00 (1H, sX2), 7.44 and 7.46 (1H, brsX2), 7.76 (1H, m), 8.21 (1H, d, J = 7.8 Hz), 8.56 (1H, d, J = 6.4 Hz) |
| 23 | $R^2$: 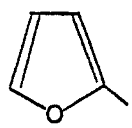 $R^4$: 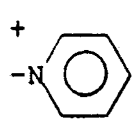 | M.p.: >200°C (decomposed)<br>IR $\nu_{max}^{nujol}$ $cm^{-1}$: 3350, 1765, 1610<br>MS (m/z): 607 ($MH^+$)<br>NMR ($D_2O$) δ : 2.92 and 2.94 (1H, dX2, J= 19 Hz and 19 Hz), 3.53 and 3.54 (1H, dX2, J = 19 Hz and 19 Hz), 5.16 (1H, brd), 5.28 (1H, d, J = 14 Hz), 5.55 - 5.63 (2H, m), 5.76 and 5.78 (1H, dX2, J = 4.9 Hz and 4.9 Hz), 6.24 - 6.30 (1H, m), 6.44 - 6.51 (1H, m), 7.01 and 7.02 (1H, sX2), 7.37 (1H, m), 8.12 (2H, m), 8.61 (1H, m), 8.96 (2H, d like) |

| | | |
|---|---|---|
| 24 | $R^2$: 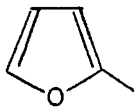 | M.p.: >200°C (decomposed)<br>IR $\nu_{max}^{nujol}$ $cm^{-1}$: 3360 (br), 3200 (sh), 1770, 1610 (br)<br>MS (m/z): 550 ($MNa^+$ - 4-methylthiazole) 528 ($MH^+$ -4-methylthiazole) |
| | $R^4$: 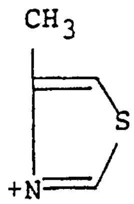 | NMR ($D_2O$) $\delta$ : 2.55 (3H, s), 3.04 and 306 (1H, dX2, J = 19 Hz and 18 Hz), 3.48 (1H, brd), 5.15 (1H, brd), 5.25 (2H, brs), 5.63 and 5.64 (1H, sX2), 5.77 and 5.80 (1H, dX2, J = 3.9 Hz and 4.4 Hz), 6.35 - 6.56 (2H, m), 7.03 (1H, s), 7.47 (1H, brs), 7.54 (1H, brs), 7.86 (1H, brs) |
| 25 | $R^2$: 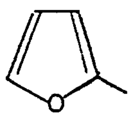 | M.p.: >200°C (decomposed)<br>IR $\nu_{max}^{nujol}$ $cm^{-1}$: 3350 (br), 3200 (sh), 1770, 1610 (br)<br>MS (m/z): 649 (free acid $+H)^+$ |
| | $R^4$: 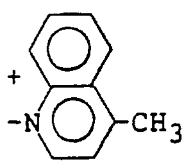 | NMR ($D_2O$) $\delta$ : 2.79 and 2.81 (1H, dX2, J = 18 Hz and 18 Hz), 3.03 (3H, s), 3.24 and 3.30 (1H, dX2, J = 18 Hz and 18 Hz), 5.07 and 5.09 (1H, dX2, J = 4.4 Hz and 4.4 Hz), 5.54 and 5.60 (1H, sX2), 5.71 and 5.72 (1H, dX2, J = 4.4 Hz and 4.4 Hz), 5.73 (1H, d, J = 16 Hz), 5.85 (1H, d, J = 16 Hz), 6.11 and 6.18 (1H, mX2), 6.36 and 6.47 (1H, dX2, J = 3.4 Hz and 3.4 Hz), 6.91 (1H, s), 7.27 (1H, brs), 7.93 (1H, d, J = 6.4 Hz), 8.02 (1H, d, J = 8.3 Hz), 8.20 (1H, t, J = 7.8 Hz), 8.46 (2H, m), 9.05 (1H, d, J = 5.9 Hz) |

| | | |
|---|---|---|
| 26 | $R^2$:<br>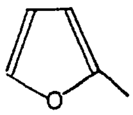<br>$R^4$:<br>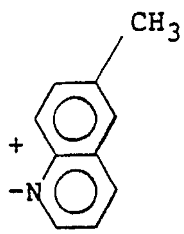 | M.p.: >170°C (decomposed)<br>IR $\nu_{max}^{nujol}$ $cm^{-1}$: 3360 (br), 3200 (sh), 1770, 1610 (br)<br>MS (m/z): 693 ($MNa^+$), (671 ($MH^+$)<br>NMR ($D_2O$) δ : 2.62 (3H, s), 2.77 and 2.81 (1H, dX2, J= 18 Hz and 18 Hz), 3.26 and 3.31 (1H, dX2, J = 18 Hz and 18 Hz), 5.07 and 5.10 (1H, dX2, J = 4.4 Hz and 4.9 Hz), 5.55 and 5.61 (1H, sX2), 5.63 (1H, m), 5.72 (2H, br), 6.10 and 6.17 (1H, mX2), 6.35 and 6.47 (1H, dX2, J = 3.4 Hz and 2.9 Hz), 6.91 (1H, s), 7.27 (1H, d, J = 1.0 Hz), 7.98 - 8.12 (3H, m), 8.37 (1H, d, J = 9.8 Hz), 9.04 (1H, d, J = 8.8 Hz), 9.16 (1H, d, J = 5.9 Hz) |
| 27 | $R^2$:<br>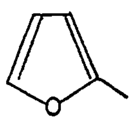<br>$R^4$:<br>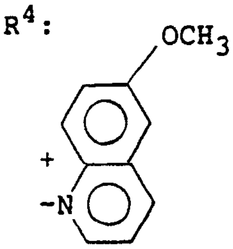 | M.p.: >200°C (decomposed)<br>IR $\nu_{max}^{nujol}$ $cm^{-1}$: 3340 (br), 3200 (sh), 1770, 1610 (br)<br>MS (m/z): 709 ($MNa^+$), 687 ($NH^+$)<br>NMR ($D_2O$) δ : 2.78 (1H, dX2, J= 18 Hz), 3.29 and 3.33 (1H, dX2, J = 18 Hz and 18 Hz), 4.02 (3H, s), 5.09 and 5.10 (1H, dX2, J = 4.4 Hz and 4.4 Hz), 5.56 and 5.60 (1H, sX2), 5.72 and 5.73 (1H; dX2, J = 4.4 Hz and 4.4 Hz), 5.84 (2H, brs), 6.13 and 6.18 (1H, mX2), 6.37 and 6.46 (1H, dX2, J = 3.4 Hz and 3.4 Hz), 6.92 (1H, s), 7.27 (1H, brs), 7.70 (1H, d, J = 2.4 Hz), 7.84 (1H, brd), 8.00 (1H, m), 8.42 and 8.44 (1H, dX2, J = 9.3 Hz and 9.3 Hz), 9.00 (1H, d, J = 8.3 Hz), 9.05 (1H, d, J = 5.9 Hz) |

| | | |
|---|---|---|
| 28 | $R^2$: 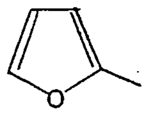 | M.p.: 200 - 210°C (decomposed)<br>IR $\nu_{max}^{nujol}$ $cm^{-1}$: 3350 (br), 3200 (sh), 1770, 1610<br>MS (m/z): 550 ($MMa^+$ - 3-bromoquinoline) 528 ($MH^+$ -3-bromoquinoline) |
| | $R^4$: 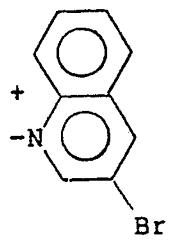 | NMR ($D_2O$) δ : 2.88 and 2.90 (1H, dX2, J= 18 Hz and 18 Hz), 3.34 and 3.38 (1H, dX2, J = 18 Hz and 18 Hz), 5.10 and 5.13 (1H, dX2, J = 4.9 Hz and 4.4 Hz), 5.57 and 5.63 (1H, sX2), 5.74 and 5.76 (1H, dX2, J = 4.4 Hz and 4.9 Hz), 5.88, 5.95 (2H, each d, J = each 14 Hz), 6.19 and 6.24 (1H, ddX2, J = 3.4, 2.0 Hz and 2.9, 2.0 Hz), 6.43 and 6.51 (1H, dX2, J = 3.4 Hz and 2.9 Hz), 6.93 and 6.94 (1H, sX2), 7.34 (1H, brs), 8.05 (1H, t, J = 7.8 Hz), 8.28 (2H, m), 8.48 (1H,brd), 9.39 (1H, brs), 9.50 (1H, d, J = 2.0 Hz) |
| 29 | $R^2$: 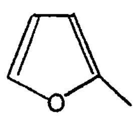 | M.p.: 190 - 200°C (decomposed)<br>IR $\nu_{max}^{nujol}$ $cm^{-1}$: 3350 (br), 3200 (sh), 1770, 1610<br>MS (m/z): 550 ($MNa^+$ -4-chloroquinoline) 528 ($MH^+$ -4-chloroquinoline) |
| | $R^4$: 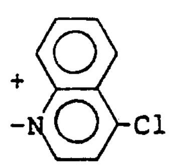 | NMR ($D_2O$) δ : 2.90 and 2.93 (1H, dX2, J = 18 Hz and 18 Hz), 3.37 and 3.41 (1H, dX2, J = 18 Hz and 18 Hz), 5.11 and 5.13 (1H, dX2, J = 4.9 Hz and 4.9 Hz), 5.59 and 5.63 (1H, sX2), 5.75 (1H, m), 5.85 (2H, brs), 6.21 and 6.26 (1H, mX2), 6.43 and 6.53 (1H, dX2, J = 2.9 Hz and 2.9 Hz), 6.95 and 6.98 (1H, sX2), 7.35 (1H, m), 8.05 - 8.33 (3H, m), 8.51 (1H, d, J = 9.3 Hz), 8.65 (1H, d, J = 8.8 Hz), 9.18 (1H, d, J = 6.3 Hz) |

| | | |
|---|---|---|
| 30 | $R^2$: 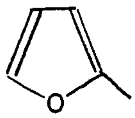 $R^4$: 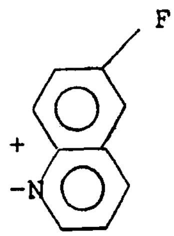 | M.p.: 190 - 200°C (decomposed)<br>IR $\nu_{max}^{nujol}$ $cm^{-1}$: 3360, 3200, 1770, 1610<br>MS (m/z): 675 ($MH^+$)<br>NMR ($D_2O$) δ : 2.86, 3.34 and 2.88, 3.39 (2H, ABqX2, J= 18 Hz), 5.12 and 5.14 (1H, dX2, J = 4.5 Hz), 5.58 and 5.63 (1H, sX2), 5.71 and 5.73 (1H, dX2, J = 4.5 Hz), 5.85 and 5.95 (2H, ABqX2, J = 15 Hz), 6.17 and 6.23 (1H, ddX2, J = 3 3 Hz and 2 Hz), 6.40 and 6.48 (1H, dX2, J = 3Hz), 6.94 and 6.95 (1H, sX2), 7.31 and 7.33 (1H, dX2, J = 2 Hz), 8.0 - 9.3 (6H, m) |
| 31 | $R^2$: 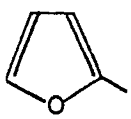 $R^4$: 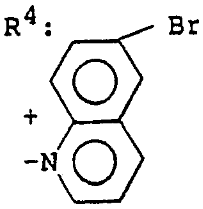 | M.p.: 195 - 210°C (decomposed)<br>IR $\nu_{max}^{nujol}$ $cm^{-1}$: 3360, 3200, 1770, 1610<br>NMR ($D_2O$) δ : 2.87, 3.38 and 2.87, 3.41 (2H, ABqX2, J = 18 Hz), 5.12 and 5.15 (1H, dX2, J = 4.5 Hz), 5.60 and 5.61 (1H, sX2), 5.76 and 5.77 (1H, dX2, J = 4.5 Hz), 5.88 (2H, sX2), 6.43 and 6.50 (1H, dX2, J = 3 Hz), 6.2 - 6.5 (1H, m), 6.98 (1H, sX2), 7.33 (1H, sX2), 8.0 - 9.3 (7H, m) |

| | | |
|---|---|---|
| 32<br>(S) | $R^2$:<br>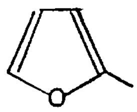 | M.p.: >170°C (decomposed)<br>IR $\nu_{max}^{nujol}$ $cm^{-1}$: 3400 - 3330, 1770, 1600, 720<br>MS (m/z): 679 ($MNa^+$), 657 ($MH^+$), 484, 309, 177, 139, 119 |
| | $R^4$:<br>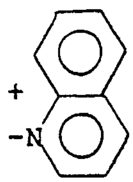 | NMR ($D_2O$) δ : 2.88 (1H, d, J= 17.1 Hz), 3.37 (1H, d, J = 18.6 Hz), 5.12 (1H, d, J = 4.9 Hz), 5.62 (1H, s), 5.76 (1H, d, J = 4.4 Hz), 5.86 (1H, d, J = 15.1 Hz), 5.95 (1H, d, 16.1 Hz), 6.19 (1H, dd, J = 2.0, 3.4 Hz), 6.48 (1H, d, J = 3.4 Hz), 6.98 (1H, s), 7.29 (1H, d, J = 1.0 Hz), 7.9 - 8.1 (2H, m), 8.1 - 8.3 (1H, m), 8.41 (1H, d, J = 8.3 Hz), 8.51 (1H, d, J = 9.3 Hz), 9.19 (1H, d, J = 8.3 Hz), 9.27 (1H, d, J = 5.9 Hz) |
| 33<br>(R) | $R^2$:<br>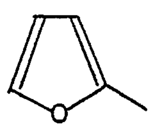 | M.p.: >170°C (decomposed)<br>IR $\nu_{max}^{nujol}$ $cm^{-1}$: 3400 - 3300, 1760, 1600, 720<br>MS (m/z): 679 ($MNa^+$), 657 ($MH^+$), 635, 177, 136 |
| | $R^4$:<br>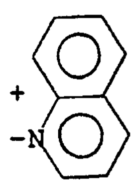 | NMR ($D_2O$) δ : 2.85 (1H, d, J= 17.6 Hz), 3.35 (1H, d, J = 18.1 Hz), 5.12 (1H, d, J = 4.4 Hz), 5.59 (1H, s), 5.75 (1H, d, J = 4.9 Hz), 5.86 (1H, d, J = 15.6 Hz), 5.96 (1H, d, J = 15.1 Hz), 6.15 (dd, J = 2.0, 3.4 Hz), 6.40 (1H, d, J = 3.4 Hz), 6.98 (1H, s), 7.30 (1H, d, J = 1.0 Hz), 7.9 - 8.1 (1H, m), 8.12 (1H, dd, J = 6.1, 8.1 Hz), 8.1 - 8.3 (1H, m), 8.41 (1H, d, J = 8.8 Hz), 8.51 (1H, d, J = 9.3 Hz), 9.20 (1H, d, J = 8.3 Hz), 9.28 (1H, d, J = 5.9 Hz) |

| | | |
|---|---|---|
| 34<br>(S) | $R^2$:<br>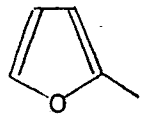<br>$R^4$:<br>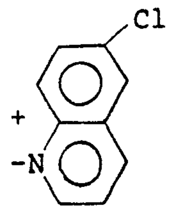 | M.p.: >170°C (decomposed)<br>IR $\nu_{max}^{nujol}$ $cm^{-1}$: 3400 - 3300, 1770, 1610, 720<br>MS (m/z): 715, 713 ($MNa^+$), 693, 691 ($MH^+$), 550, 484, 177, 139<br>NMR ($D_2O$) δ : 2.89 (1H, d, J= 17.6 Hz), 3.42 (1H, d, J = 18.1 Hz), 5.14 (1H, d, J = 4.9 Hz), 5.63 (1H, s), 5.77 (1H, d, J = 4.4 Hz), 5.89 (1H, s), 6.2 - 6.3 (1H, m), 6.4 - 6.5 (1H, m), 6.95 (1H, s), 7.3 - 7.4 (1H, m), 8.12 (1H, dd, J = 6.1, 8.5 Hz), 8.22 (1H, brd, J = 9.3 Hz), 8.42 (1H, brs), 8.53 (1H, d, J = 9.3 Hz), 9.10 (1H, d, J = 8.8 Hz), 9.26 (1H, d, J = 5.4 Hz) |
| 35<br>(R) | $R^2$:<br>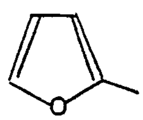<br>$R^4$:<br>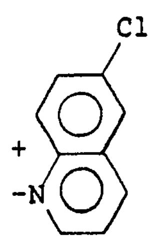 | M.p.: >170°C (decomposed)<br>IR $\nu_{max}^{nujol}$ $cm^{-1}$: 3400 - 3300, 1760, 1610, 720<br>MS (m/z): 715, 713 ($MNa^+$), 693, 691 ($MH^+$), 550, 484, 177, 152<br>NMR ($D_2O$) δ : 2.86 (1H, d, J= 17.6 Hz), 3.36 (1H, d, J = 17.6 Hz), 5.12 (1H, d, J = 4.9 Hz), 5.59 (1H, s), 5.75 (1H, d, J = 4.4 Hz), 5.88 (2H, brs), 6.21 (1H, dd, J = 1.5, 3.4 Hz), 6.44 (1H, d, J = 3.4 Hz), 6.94 (1H, s), 7.35 (1H, brs), 8.14 (1H, dd, J = 6.4, 8.3 Hz), 8.21 (1H, brd, J = 9.8 Hz), 8.41 (1H, brs), 9.11 (1H, d, J = 8.3 Hz), 9.26 (1H, d, J = 5.4 Hz) |

| | | |
|---|---|---|
| 36 | $R^2$:<br>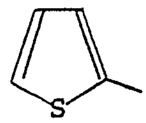<br>$R^4$:<br>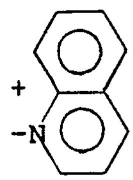 | M.p.: >160°C (decomposed)<br>IR $\nu_{max}^{nujol}$ $cm^{-1}$: 3280, 3200, 1760, 1610<br>MS (m/z): 673 ($MH^+$)<br>NMR ($D_2O$) δ : 2.76 and 2.80 (1H, dX2, J= 17.6 Hz and 17.6 Hz), 3.24 and 3.30 (1H, dX2, J = 17.6 Hz and 17.6 Hz), 5.09 and 5.06 (1H, dX2, J = 4.9 Hz and 4.9 Hz), 5.75 (2H, m), 6.80 (1H, d, J = 16 Hz), 6.90 (1H, d, J = 16 Hz), 6.70 - 7.28 (3H, m), 6.89 and 6.90 (1H, sX2), 8.00 (2H, m), 8.20 (1H, dd), 8.34 (1H, d), 8.45 (1H, d), 9.14 (1H, d), 9.23 (1H, d) |
| 37 | $R^2$:<br>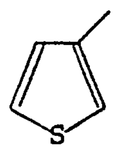<br>$R^4$:<br>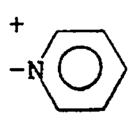 | M.p.: >200°C (decomposed)<br>IR $\nu_{max}^{nujol}$ $cm^{-1}$: 3325, 3200, 1770, 1610<br>MS (m/z): 645 ($MNa^+$), 623 ($MH^+$)<br>NMR ($D_2O$) δ : 2.88 and 2.94 (1H, dX2, J= 18 Hz and 18 Hz), 3.50 and 3.53 (1H, dX2, J = 18 Hz and 18 Hz), 5.14 and 5.17 (1H, dX2, J = 4.4 Hz and 4.4 Hz), 5.25 (1H, d, J = 15 Hz), 5.57 and 5.59 (1H, dX2, J = 15 Hz and 15 Hz), 5.62 (1H, s), 5.74 and 5.77 (1H, dX2, J = 4.4 Hz and 4.4 Hz), 7.00 and 7.01 (1H, sX2), 7.12 - 7.48 (3H, m), 8.07 - 8.17 (2H, m), 8.60 (1H, m), 8.95 and 8.98 (2H, dX2, J = 5.4 Hz and 5.9 Hz) |

| | | |
|---|---|---|
| 38 | $R^2$: 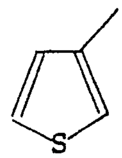 <br> $R^4$: 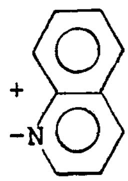 | M.p.: >200°C (decomposed)<br>IR $\nu_{max}^{nujol}$ $cm^{-1}$: 3355, 3200, 1770, 1600<br>MS (m/z): 695 ($MNa^+$), 673 ($MH^+$)<br>NMR ($D_2O$) δ : 2.75 and 2.82 (1H, dX2, J= 18 and 18 Hz), 3.27 and 3.32 (1H, dX2, J = 18 Hz and 18 Hz), 5.08 and 5.10 (1H, dX2, J = 4.9 Hz and 4.9 Hz), 5.57 and 5.61 (1H, sX2), 5.71 and 5.73 (1H, dX2, J = 4.9 Hz and 4.9 Hz), 5.87 (2H, brs), 6.92 and 6.93 (1H, sX2), 7.14 - 7.45 (3H, m), 7.97 - 8.52 (5H, m), 9.15 (1H, brd), 9.23 and 9.25 (1H, dX2, J = 4.9 Hz and 4.9 Hz) |

Example 39

A mixture of 7β-{(Z)-2-(2-aminothiazol-4-yl)-2-[(2-thienyl)(carboxy)methyloxyimino]-acetamido}cephalosporanic acid disodium salt (0.81 g), 1-((thiocarbamoyl)methyl)-4-thiopyridone(0.24 g), sodium iodide (1.8 g), acetonitrile (10 ml) and water (12 ml) is stirred at 65 - 70° C under argon atmosphere for 4 hours. The mixture is concentrated under reduced pressure to remove acetonitrile. The residue is basified with aqueous sodium hydrogen carbonate solution. The mixture is purified by HP-20 column chromatography (solvent: 10 - 20 % aqueous methanol solution). The initial part, the middle part and the last part of the desired fractions are concentrated respectively under reduced pressure. The each concentrated fraction is lyophilized respectively to give α-isomer, a mixture of α-isomer and β-isomer and β-isomer of 7β-{(Z)-2-(2-aminothiazol-4-yl)-2-[(2-thienyl)(carboxy)methyloxyimino]acetamido}-3-[[1-(thiocarbamoylmethyl)-4-pyridinio]thiomethyl]-3-cephem-4-carboxylate sodium salt respectively.

α-Isomer:

Yield: 0.1 g
M.p.: 180 - 183° C (decomposed)

$$IR\ \nu_{max}^{nujol} cm^{-1}: 3400 - 3100, 1760, 1630, 1600$$

MS (m/z): 728 ($MH^+$)
NMR ($D_2O$) δ : 3.25 (1H, d, J = 17 Hz), 3.53 (1H, d, J = 17 Hz), 4.25 (2H, s), 5.08 (1H, d, J = 4.4 Hz), 5.36 (2H, s), 5.64 (1H, d, J = 4.4 Hz), 5.79 (1H, s), 6.93 (1H, s), 7.00 (1H, t), 7.22 (1H, d, J = 3 Hz), 7.41 (1H, d, J = 4.4 Hz), 7.81 (2H, d, J = 6.8 Hz), 8.24 (2H, d, J = 6.8 Hz)

A mixture of α- and β-isomers:

Yield: 0.25 g

M.p.: >170° C (decomposed)

$$IR\ \nu_{max}^{nujol} cm^{-1}: 3400 - 3100, 1760, 1630, 1600$$

MS (m/z): 728 ($MH^+$)
NMR ($D_2O$) δ : 3.18 and 3.25 (1H, dX2, J = 18 Hz and 18 Hz), 3.48 and 3.53 (1H, dX2, J = 17 Hz and 17 Hz), 4.25 (2H, s), 5.05 and 5.98 (1H, dX2, J = 4.4 Hz and 4.4 Hz), 5.37 (2H, s), 5.60 and 5.63 (1H, dX2, J = 4.4 Hz and 4.4 Hz), 5.76 and 5.79 (1H, sX2), 6.93 and 6.94 (1H, sX2), 6.99 (1H, dd), 7.20 (1H, dd), 7.40 (1H, dd), 7.82 (2H, d), 8.25 (2H, m)

β-Isomer:

Yield: 0.05 g
M.p.: >170° C (decomposed)

$$IR\ \nu_{max}^{nujol} cm^{-1}: 3400 - 3100, 1760, 1630, 1600$$

MS (m/z): 728 ($MH^+$)
NMR ($D_2O$) δ : 3.18 (1H, d, J = 18 Hz), 3.48 (1H, d, J = 17 Hz), 4.25 (2H, s), 5.05 (1H, d, J = 4.4 Hz), 5.37 (2H, s), 5.60 (2H, d, J = 4.4 Hz), 5.76 (1H, s), 6.94 (1H, s), 6.98 (1H, t), 7.19 (1H, d, J = 3.4 Hz), 7.39 (1H, d, J = 4.9 Hz), 7.82 (2H, d, J = 6.1 Hz), 8.27 (2H, d, J = 6.1 Hz)

Example 40

To a solution of sodium hydrogen carbonate (0.24 g) in water (25 ml) are added 7β-{(Z)-2-(2-aminothiazol-4-yl)-2-[(2-furyl)(carboxy)methyloxyimino]acetamido}cephalosporanic acid (0.79 g), acetonitrile (15 ml), 1-benzyl-4-thiopyridone (0.38 g) and sodium iodide (2.10 g) and stirred. After adjusting the pH value of the mixture to pH 6.2 with acetic acid, the mixture is stirred at 65 - 70° C under argon atmosphere for 7 hours. The mixture is concentrated and the solvent is distilled off, and the insoluble materials are filtered off, and further the filtrate is concentrated. The pH value of the residue is adjusted to pH 8 with sodium hydrogen carbonate, and then, it is purified by HP-20 column chromatography (solvent: 20 -30 % aqueous methanol solution). After the purification, the solvent is distilled off under reduced pressure and lyophilized to give 7β-{(Z)-2-(2-aminothiazol-4-yl)-2-[(2-furyl)(carboxy)methyloxyimino]acetamido}-3-[(1-benzyl-4-pyridinio)thiomethyl]-3-cephem-4-carboxylate sodium salt (0.48 g) as powder.
M.p.: >170° C (decomposed).

$$IR\ \nu_{max}^{nujol} cm^{-1}\ 3350, 1760, 1620, 1600$$

MS (m/z): 751 ($MNa^+$), 729 ($MH^+$)
NMR ($D_2O$) δ : 3.23 and 3.25 (1H, dX2, J = 17 Hz and 17 Hz), 3.51 and 3.54 (1H, dX2, J = 17 Hz and 17 Hz), 4.20 (1H, d, J = 14 Hz), 4.27 and 4.30 (1H, dX2, J = 14 Hz and 14 Hz), 5.01 and 5.03 (1H, dX2, J = 4.9 Hz and 4.4 Hz), 5.57 - 5.63 (4H, m), 6.37 (1H, m), 6.48 (1H, m), 6.94 (1H, s), 7.44 (6H, brs), 7.81 (2H, brd), 8.46 (2H, d, J = 7.3 Hz)

Example 41

7β-{(Z)-2-(2-Aminothiazol-4-yl)-2-[(2-thienyl)(carboxy)methyloxyimino]acetamido}cephalosporanic acid and 1-methyl-2-thiopyridone are treated in the same manners as in Example 40 to give 7β-{(Z)-2-(2-aminothiazol-4-yl)-2-[(2- thienyl)(carboxy)methyloxyimino]acetamido}-3-[(1-methyl-2-pyridinio)thiomethyl]-3-cephem-4-carboxylate sodium salt.

M.p.: 180 - 190 °C (decomposed)

$$IR\ \nu_{max}^{nujol} cm^{-1}: 3400 - 3100, 1760, 1600$$

MS (m/z): 669 ($MH^+$)

NMR ($D_2O$) $\delta$ : 3.27 and 3.29 (1H, dX2, J = 17.3 Hz and 17 Hz), 3.56 and 3.58 (1H, dX2, J = 17.3 Hz and 17 Hz), 4.25 (1H, d, J = 13.7 Hz), 4.41 (1H, d, J = 13.7 Hz), 5.05 and 5.06 (1H, dX2, J = 4.4 Hz and 4.4 Hz), 5.64 and 5.67 (1H, dX2, J = 4.4 Hz and 4.4 Hz), 5.78 (1H, s), 7.01 (1H, s), 7.06 (1H, d, d, J = 4.9 Hz, J = 3.4 Hz), 7.23 (1H, d, J = 3.4 Hz), 7.48 (1H, d, J = 4.9 Hz), 7.68 (1H, t), 7.89 (1H, d, J = 8.8 Hz), 8.28 (1H, m), 8.64 (1H, d, J = 6.3 Hz)

Examples 42 - 60

7β-{(Z)-2-(2-Aminothiazol-4-yl)-2-[(2-thienyl or furyl)(carboxy)methyloxyimino]-acetamido}cephalosporanic acid and pyridone compound are treated in the same manners as in Examples 39 or 40 to give the compounds of the following table 2.

Table 2

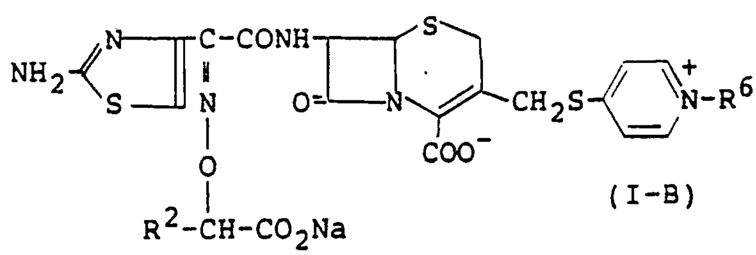

(I-B)

wherein the compound (I-B) is having Z-configuration.

| Ex. No. | $R^2$ and $R^6$ | Compound (I-B)<br>Physicochemical properties |
|---|---|---|
| 42 | $R^2$:<br>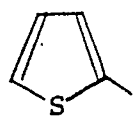<br>$R^6$:<br>$-CH_2-CH=CH_2$ | M.p.: >150°C (decomposed)<br>MS (m/z): 695 ($MH^+$)<br>NMR ($D_2O$) δ : 3.25 and 3.29 (1H, dX2, J= 17.6 Hz and 17.6 Hz), 3.53 and 3.57 (1H, dX2, J = 17.6 Hz and 17.6 Hz), 4.26 (2H, s), 4.96 (2H, d, J = 5.8 Hz), 5.07 and 5.10 (1H, dX2, J = 4.9 Hz and 4.9 Hz), 5.40 (1H, d, J = 17.1 Hz), 5.49 (1H, d, J = 10.2 Hz), 5.61 and 5.63 (1H, dX2, J = 4.9 Hz and 4.9 Hz), 5.75 and 5.78 (1H, sX2), 6.04 (1H, m), 6.93 and 6.95 (1H, sX2), 7.02 (1H, d, d, J = 8.8 Hz, J= 4.4 Hz), 7.21 (1H, m), 7.44 (1H, m), 7.84 (2H, d, d, J= 7.3 Hz, J = 2.3 Hz), 8.35 (2H, d, d, J = 7.3 Hz, J = 2.3 Hz) |
| 43 | $R^2$:<br>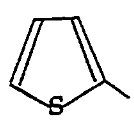<br>$R^6$:<br>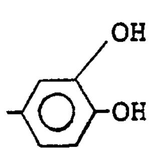 | M.p.: 190 - 200°C (decomposed)<br>IR $\nu_{max}^{nujol}$ $cm^{-1}$: 3400 - 3000, 1760, 1610,<br>MS (m/z): 763 ($MH^+$)<br>NMR ($DMSO-d_6+D_2O$) δ : 3.15 and 3.36 (1H, dX2, J= 17.1 Hz and 17.1 Hz), 3.30 and 3.52 (1H, dX2, J = 17.1 Hz and 17.1 Hz), 4.26 (1H, t), 4.73 (1H, d), 4.95 and 5.05 (1H, dX2, J = 4.9 Hz and 4.9 Hz), 5.46 and 5.61 (1H, dX2, J = 4.9 Hz and 4.9 Hz), 5.49 (1H, s), 6.76 - 7.40 (7H, m), 8.38 (2H, m), 8.70 (2H, m) |

| | | |
|---|---|---|
| 44 | $R^2$: 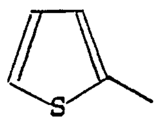 | M.p.: 180 - 185°C (decomposed)<br>IR $\nu_{max}^{nujol}$ $cm^{-1}$: 3400, 1760, 1650, 1620, 1600<br>MS (m/z): 737 ($MNa^+$)<br>NMR ($D_2O$) $\delta$ : 2.12 (3H, s), 3.25 and 3.28 (1H, dX2, J= 17.6 Hz and 17.6 Hz), 3.53 and 3.57 (1H, dX2, J = 17.6 Hz and 17.6 Hz), 4.27 (2H, s), 5.07 and 5.09 (1H, dX2, J = 4.4 Hz and 4.4 Hz), 5.43 (2H, s), 5.62 and 5.64 (1H, dX2, J = 4.4 and 4.4 Hz), 5.75 and 5.77 (1H, sX2), 6.95 and 6.96 (1H, sX2), 7.00 (1H, m), 7.22 (1H, d, d, J = 7.3 Hz, J = 2.9 Hz), 7.44 (1H, m), 7.87 (1H, d, d, J = 7.3 Hz, J = 2.2 Hz), 8.54 (1H, d, d, J = 7.3 Hz, J = 2.2 Hz) |
| | $R^6$: $-CH_2SCH_3$ | |
| 45 | $R^2$: 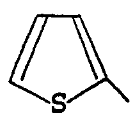 | M.p.: >180°C (decomposed)<br>IR $\nu_{max}^{nujol}$ $cm^{-1}$: 3350, 3190, 1760, 1630, 1600<br>MS (m/z): 669 ($MH^+$)<br>NMR ($D_2O$) $\delta$ : 3.27 and 3.31 (1H, dX2, J= 17.6 Hz and 17.6 Hz), 3.54 and 3.58 (1H, dX2, J = 17.6 Hz and 17.6 Hz), 4.13 (3H, s), 4.23 (2H, s), 5.07 and 5.10 (1H, dX2, J = 4.6 Hz and 4.6 Hz), 5.61 and 5.63 (1H, dX2, J = 4.6 Hz and 4.6 Hz), 5.76 and 5.78 (1H, sX2), 6.91 and 6.92 (1H, sX2), 7.00 (1H, m), 7.20 (1H, m), 7.45 (1H, m), 7.78 and 7.80 (2H, dX2), 8.27 (2H, d) |
| | $R^6$: $-CH_3$ | |

| | | |
|---|---|---|
| 46 | $R^2$: 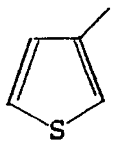 $R^6$: $-CH_2CSNH_2$ | M.p.: 190 - 200°C (decomposed)<br>IR $\nu_{max}^{nujol}$ $cm^{-1}$: 3320, 3200, 3120, 1760, 1630, 1600<br>MS (m/z): 728 ($MH^+$)<br>NMR ($D_2O$) δ : 3.20 and 3.23 (1H, dX2, J= 18 Hz and 18 Hz), 3.49 and 3.52 (1H, dX2, J = 18 Hz and 18 Hz), 4.27 (2H, brs), 5.04 and 5.07 (1H, dX2, J = 4.4 Hz and 4.9 Hz), 5.41 (2H, brs), 5.62 - 5.64 (2H, m), 6.97 and 6.98 (1H, sX2), 7.15 - 7.21 (1H, m), 7.33 - 7.49 (2H, m), 7.85 (2H, d, J = 6.8 Hz), 8.32 (2H, d, J = 6.8 Hz) |
| 47 | $R^2$: 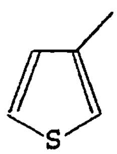 $R^6$: $-CH_3$ | M.p.: 180 - 190°C (decomposed)<br>IR $\nu_{max}^{nujol}$ $cm^{-1}$: 3340, 3200, 3120, 1765, 1630, 1600<br>MS (m/z): 691 ($MNa^+$), 669 ($MH^+$)<br>NMR ($D_2O$) δ : 3.27 and 3.28 (1H, dX2, J= 18 Hz and 18 Hz), 3.53 and 3.57 (1H, dX2, J = 18 Hz and 18 Hz), 4.05 - 4.40 (2H, m), 4.14 (3H, s), 5.06 and 5.08 (1H, dX2, J = 4.9 Hz and 4.4 Hz), 5.62 and 5.63 (1H, sX2), 5.67 and 5.75 (1H, dX2, J = 4.9 Hz and 4.4 Hz), 6.96 (1H, s), 7.15 - 7.22 (1H, m), 7.36 - 7.54 (2H, m), 7.79 (2H, m), 8.32 (2H, d, J = 6.4 Hz) |

| | | |
|---|---|---|
| 48 | $R^2$:<br>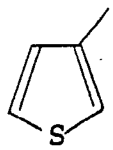<br>$R^6$:<br>$-CH_2SCH_3$ | M.p.: >180°C (decomposed)<br>IR $\nu_{max}^{nujol}$ $cm^{-1}$: 3350, 3200, 3115, 1765, 1625, 1600<br>MS (m/z): 737 ($MNa^+$), 715 ($MH^+$)<br>NMR ($D_2O$) δ : 2.11 and 2.12 (3H, sX2), 3.27 and 3.29 (1H, dX2, J = 18 Hz and 18 Hz), 3.53 and 3.57 (1H, dX2, J = 18 Hz and 18 Hz), 4.28 (2H, brs), 5.07 and 5.09 (1H, dX2, J = 4.4 Hz and 4.4 Hz), 5.44 (2H, s), 5.62 - 5.70 (2H, m), 6.96 and 6.97 (1H, sX2), 7.16 - 7.22 (1H, m), 7.36 - 7.43 (1H, m), 7.48 - 7.52 (1H, m), 7.85 - 7.90 (2H, m), 8.55 (2H, brd) |
| 49 | $R^2$:<br>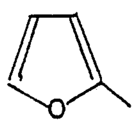<br>$R^6$:<br>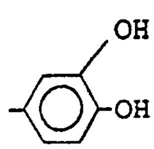 | M.p.: >200°C (decomposed)<br>IR $\nu_{max}^{nujol}$ $cm^{-1}$: 3350, 1760, 1620<br>MS (m/z): 747 ($MH^+$)<br>NMR ($DMSO-d_6+D_2O$) δ : 4.30 (1H, brd), 4.72 (1H, brd), 4.93 and 5.02 (1H, dX2, J = 4.9 Hz and 4.9 Hz), 5.32 and 5.33 (1H, sX2), 5.50 and 5.61 (1H, dX2, J = 4.9 Hz and 4.9 Hz), 6.26 - 6.43 (2H, m), 6.78 (1H, s), 6.87 (3H, br), 7.46 and 7.56 (1H, brX2), 8.35 (2H, br), 8.71 (2H, br) |

| | | |
|---|---|---|
| 50 | $R^2$:<br>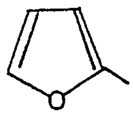<br>$R^6$:<br>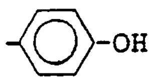 | M.p.: >180°C (decomposed)<br>IR $\nu_{max}^{nujol}$ $cm^{-1}$: 3340 - 3100, 1760, 1620<br>MS (m/z): 753 ($MNa^+$), 731 ($MH^+$)<br>NMR ($D_2O$) δ : 3.30 and 3.34 (1H, dX2, J= 18 Hz and 18 Hz), 3.58 and 3.64 (1H, dX2, J = 18 Hz and 18 Hz), 4.22 - 4.42 (2H, m), 5.07 and 5.11 (1H, dX2, J = 4.9 Hz and 4.9 Hz), 5.60 (1H, s), 5.63 (1H, d, J = 4.9 Hz), 6.45 (1H, m), 6.52 (1H, m), 6.92 and 6.93 (1H, sX2), 7.03 (2H, d, J = 8.8 Hz), 7.47 (2H, d, J = 8.8 Hz), 7.49 (1H, brs), 7.93 (2H, t), 8.53 (2H, d, J = 6.8 Hz) |
| 51 | $R^2$:<br>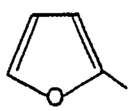<br>$R^6$:<br>$-CH_2CH_2$<br>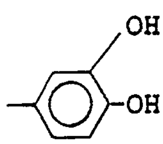 | M.p.: >180°C (decomposed)<br>IR $\nu_{max}^{nujol}$ $cm^{-1}$: 3350, 1760, 1620<br>MS (m/z): 775 ($MH^+$)<br>NMR (DMSO-$d_6$) δ : 2.95 (2H, br), 4.03 - 4.85 (4H, m), 4.95 and 5.04 (1H, dX2, J = 4.9 Hz and 4.9 Hz), 5.32 (1H, s), 5.52 and 5.65 (1H, ddX2, J = 8.7, 4.9 Hz and 8.7, 4.9 Hz), 6.17 - 6.62 (5H, m), 6.79 and 6.86 (1H, sX2), 7.18 (2H, br), 7.53 (1H, d), 8.16 and 8.24 (2H, dX2, J = 6.4 Hz and 6.4 Hz), 8.56 and 8.66 (2H, dX2, J = 6.4 Hz and 6.4 Hz), 11.6 and 11.8 (1H, dX2, J = 8.7 Hz and 8.7 Hz) |

| | | |
|---|---|---|
| 52 | $R^2$:<br>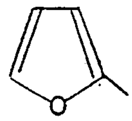 | M.p.: >170°C (decomposed)<br>IR $\nu_{max}^{nujol}$ $cm^{-1}$: 3350, 1760, 1620, 1600<br>MS (m/z): 699 ($MH^+$) |
| | $R^6$:<br>$-CH_2SCH_3$ | NMR ($D_2O$) δ : 2.13 (3H, s), 3.29 (1H, d, J= 17 Hz), 3.57 and 3.60 (1H, dX2, J = 17 Hz and 17 Hz), 4.20 and 4.22 (1H, dX2, J = 14 Hz and 14 Hz), 4.33 (1H, d, J = 14 Hz), 5.07 and 5.08 (1H, dX2, J = 4.9 Hz and 4.9 Hz), 5.46 (2H, s), 5.61 - 5.68 (2H, m), 6.42 - 6.55 (2H, m), 6.99 (1H, s), 7.50 (1H, br), 7.87 (2H, brd), 8.58 (2H, d, J = 6.8 Hz) |
| 53 | $R^2$:<br>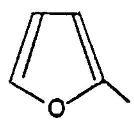 | M.p.: ~ 190°C (decomposed)<br>IR $\nu_{max}^{nujol}$ $cm^{-1}$: 3300 - 3100, 1760, 1620, 1600<br>MS (m/z): 712 ($MH^+$) |
| | $R^6$:<br>$-CH_2CSNH_2$ | NMR (DMSO-$d_6$) δ : 4.17 and 4.36 (1H, dX2, J= 14 Hz and 13 Hz), 4.62 and 4.76 (1H, dX2, J = 13 Hz and 14 Hz), 4.95 and 5.05 (1H, dX2, J = 4.9 Hz and 4.9 Hz), 5.29 and 5.30 (1H, sX2), 5.48 - 5.65 (3H, m), 6.26 - 6.40 (2H, m), 6.76 and 6.86 (1H, sX2), 7.18 (2H, brs), 7.49 and 7.57 (1H, brX2), 8.25 and 8.42 (2H, dX2, J = 6.8 Hz and 6.8 Hz), 8.52 (2H, m), 11.6 and 11.7 (1H, dX2, J = 7.8 Hz and 8.8 Hz) |

| | | |
|---|---|---|
| 54 | $R^2$:<br>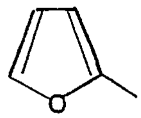<br>$R^6$:<br>$-CH_2CSNHCH_3$ | M.p.: >180°C (decomposed)<br>IR $\nu_{max}^{nujol}$ $cm^{-1}$: 3300, 1760, 1620, 1600<br>MS (m/z): 726 ($MH^+$)<br>NMR ($D_2O$) δ : 3.15 (3H, s), 3.27 (1H, brd), 3.55 and 3.58 (1H, dX2, J = 18 Hz), 4.20 - 4.38 (2H, m), 5.07 and 5.08 (1H, dX2, J = 4.4 Hz), 5.41 (2H, s), 5.62 - 5.68 (2H, m), 6.44 (1H, m), 6.53 (1H, m), 6.988 and 6.992 (1H, sX2), 7.48 (1H, m), 7.87 and 7.89 (2H, dX2, J = 6.8 Hz), 8.36 (2H, d, J = 6.8 Hz) |
| 55 | $R^2$:<br>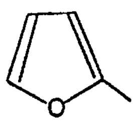<br>$R^6$:<br>$-CH_3$ | M.p.: >180°C (decomposed)<br>IR $\nu_{max}^{nujol}$ $cm^{-1}$: 3360, 1760, 1630, 1600<br>MS (m/z): 653 ($MH^+$)<br>NMR ($D_2O$) δ : 3.30 (1H, d, J= 18 Hz), 3.57 and 3.60 (1H, dX2, J = 18 Hz and 18 Hz), 4.13 - 4.35 (2H, m), 4.16 (3H, s), 5.07 (1H, d, J = 4.4 Hz), 5.61 - 5.68 (2H, m), 6.42 - 6.56 (2H, m), 6.96 and 6.97 (1H, sX2), 7.51 (1H, brs), 7.81 and 7.82 (2H, dX2, J = 6.8 Hz and 6.8 Hz), 8.34 (2H, d, J = 6.8 Hz) |

| | | |
|---|---|---|
| 56 | $R^2$: 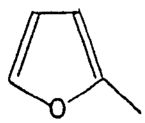<br>$R^6$: $-CH_2CH=CH_2$ | M.p.: >180°C (decomposed)<br>IR $\nu_{max}^{nujol}$ $cm^{-1}$: 3400 - 3100, 1765, 1625, 1600<br>MS (m/z): 701 ($MNa^+$), 679 ($MH^+$)<br>NMR ($D_2O$) δ : 3.29 (1H, d, J= 18 Hz), 3.57 and 3.60 (1H, dX2, J = 18 Hz and 18 Hz), 4.21 and 4.24 (1H, dX2, J = 14 Hz and 14 Hz), 4.32 (1H, d, J = 14 Hz), 5.00 (2H, d, J = 6.4 Hz), 5.07 and 5.08 (1H, dX2, J = 4.4 Hz and 4.9 Hz), 5.41 (1H, d, J = 17 Hz), 5.50 (1H, d, J = 10 Hz), 5.61 and 5.63 (1H, sX2), 5.65 and 5.67 (1H, dX2, J = 4.4 Hz and 4.9 Hz), 5.98 - 6.18 (1H, m), 6.42.- 6.46 (1H, m), 6.49 and 6.54 (1H, dX2, J = 2.9 Hz and 2.9 Hz), 6.98 (1H, s), 7.50 (1H, brs), 7.85 (2H, d like), 8.40 (2H, d, J = 6.8 Hz) |
| 57 | $R^2$: 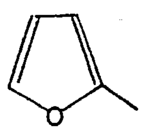<br>$R^6$: $-(CH_2)_3CNH_2$ (C=S) | M.p.: >180°C (decomposed)<br>IR $\nu_{max}^{nujol}$ $cm^{-1}$: 3300 - 3100, 1760, 1620, 1600<br>MS (m/z): 762 ($MNa^+$), 740 ($MH^+$)<br>NMR ($D_2O$) δ : 2.30 - 2.45 (2H, m), 2.59 - 2.74 (2H, m), 3.29 (1H, d, J = 19 Hz), 3.56 and 3.59 (1H, dX2, J = 19Hz and 19 Hz), 4.18 - 4.70 (4H, m), 5.08 (1H, br), 5.63 (2H, m), 6.45 - 6.55 (2H, m), 6.99 (1H, s), 7.51 (1H, brs), 7.85 (2H, d like), 8.44 (2H, d like) |

| | | |
|---|---|---|
| 58 | $R^2$:<br>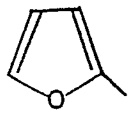<br>$R^6$:<br>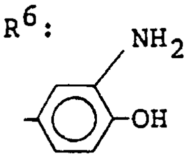 | M.p.: >200°C (decomposed)<br>IR $\nu_{max}^{nujol}$ $cm^{-1}$: 3350, 1760, 1620<br>MS (m/z): 768 ($MNa^+$), 746 ($M^+$)<br>NMR ($D_2O$) δ : 3.15 - 3.64 (2H, m), 4.14 - 4.40 (2H, m), 5.06 and 5.09 (1H, dX2, J = 4.9 Hz and 4.9 Hz), 5.57 - 5.64 (2H, m), 6.40 - 6.53 (2H, m), 6.80 - 6.94 (3H, m), 7.50 (1H, brs), 7.86 (2H, br), 8.46 (2H, d like) |
| 59 | $R^2$:<br>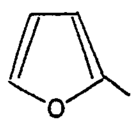<br>$R^6$:<br>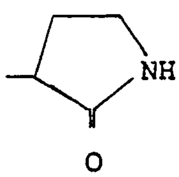 | M.p.: >180°C (decomposed)<br>IR $\nu_{max}^{nujol}$ $cm^{-1}$: 3400 - 3100, 1770, 1710, 1650, 1630, 1600<br>MS (m/z): 744 ($MNa^+$), 722 ($MH^+$)<br>NMR ($D_2O$) δ : 2.45 - 2.65 (1H, m), 2.85 - 3.05 (1H, m), 3.28 (1H, d, J = 17 Hz), 3.55 - 3.63 (3H, m), 4.24 (1H, d, J = 14 Hz), 4.35 and 4.37 (1H, dX2, J = 14 Hz and 14 Hz), 5.07 (1H, d like), 5.49 - 5.69 (3H, m), 6.44 (1H, br), 6.53 (1H, m), 7.00 (1H, s), 7.50 (1H, s), 7.91 (2H, d, J = 6.4 Hz), 8.49 (2H, d, J = 6.4 Hz) |

| | | |
|---|---|---|
| 60 | $R^2$: 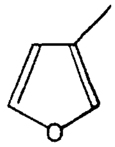 | m.p.: 190 - 198°C (decomposed) |
| | $R^6$: 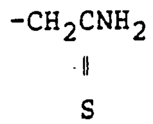 | IR $\nu_{max}^{nujol}$ $cm^{-1}$: 3300, 3200, 1760, 1620<br>NMR ($D_2O$) δ : 3.32 and 3.35 (1H, dX2, J= 17.1 Hz and 17.1 Hz), 3.58 and 3.61 (1H, dX2, J = 17.1 Hz and 17.1 Hz), 4.24 (1H, d, J = 14.2 Hz), 4.35 (1H, d, J = 14.2 Hz), 5.11 and 5.12 (1H, dX2, J = 4.4 Hz and 4.9 Hz), 5.33 and 5.41 (2H, sX2), 5.52 and 5.53 (1H, sX2), 5.66 and 5.69 (1H, dX2, J = 4.4 Hz and 4.9 Hz), 6.55 (1H, d, d), 6.96 (1H, s), 7.47 (1H, d, d,), 7.64 (1H, m), 7.83 and 7.85 (2H, dX2), 8.33 (2H, d, J = 6.8 Hz) |

Reference Example 1

(1) A suspension of N-hydroxyphthalimide potassium salt (31.1 g) in dimethylformamide (250 ml) is cooled under argon atmosphere to a temperature below 0°C, and to this solution is added dropwise a solution of 2-bromo-2-(2-thienyl)acetic acid tertiary butyl ester (33.0 g) in dimethylformamide (50 ml) at 4°C. The mixture is stirred at the same temperature for 1 hour. The mixture is poured into ice-water and extracted with ethyl acetate. The extract is washed with aqueous sodium hydrogen carbonate solution and water, then dried. After the solution is filtered, the solvent is distilled off under reduced pressure to give 2-phthalimidoxy-2-(2-tienyl)acetic acid tertiary butyl ester (41.4 g,) as a yellow brown oily product.
Yield: 97 %

$$IR\ \nu_{max}^{nujol}\ cm^{-1}: 1790, 1740, 1610$$

MS (m/z): 286 ($M^+$ -$C_4H_9O$)

(2) A solution of this product (41.3 g) in methylene chloride (300 ml) is cooled under argon atmosphere to -50°C and thereto added dropwise methylhydrazine (5.3 g), and the mixture is stirred at -50°C to 0°C for for 1 hour, and further stirred at 0 to 5°C for 20 minutes. The mixture is filtered and the filtrate is washed with ice-water and then dried. After the solution is filtered, the solvent is distilled off under reduced pressure. The residue is purified by silica gel chromatography (solvent: hexane/ethyl acetate) to give 2-aminoxy-2-(2-tienyl)acetic acid tertiary butyl ester (24.5 g) as light yellow oily product.
Yield: 93 %

$$IR\ \nu_{max}^{nujol}\ cm^{-1}: 3330, 3260, 3110, 1740, 1580$$

MS (m/z): 197 ($M^+$ -32)

(3) The product (127 mg) and 2-tritylaminothiazol-4-yl-glyoxylic acid (200 mg) are dissolved in a mixture of acetonitrile (2 ml), tetrahydrofuran (1 ml) and water (2.6 ml). The pH value of the mixture is adjusted to

pH 5.0 with aqueous sodium hydrogen carbonate solution and then the mixture is stirred at room temperature for 22 hours. After the pH value of the mixture is adjusted to pH 7.85 with aqueous sodium hydrogen carbonate solution, the solvent is distilled off under reduced pressure. The pH value of the residue is adjusted to pH 1.98 with 10 % hydrochloric acid and extracted with ethyl acetate. The extract is washed with saline solution, dried and then therefrom the solvent is distilled off. The residue is treated with isopropyl ether to give 2-(2-tritylaminothiazol-4-yl)-2-[(2-thienyl)-(tert.-butoxycarbonyl)methyloxyimino]acetic acid (256 mg) as powder.
Yield: 89 %

$$IR\ \nu_{max}^{nujol} cm^{-1}: 3240,\ 2800 - 2200,\ 1740$$

MS (m/z): 626 ($MH^+$)

Reference Example 2

(1) To a solution of (2-furyl)glyoxylic acid (7 g) in ether (100 ml) is added diphenyldiazomethane (19.4 g) and the mixture is stirred at room temperature for 2 days. The mixture is concentrated under reduced pressure and the residue is purified by silica gel chromatography (solvent: hexane/ethyl acetate) to give (2-furyl)glyoxylic acid diphenylmethyl ester (14.7 g) as light yellow crystal.
Yield: 96 %
M.p.: 33 - 34 ° C

$$IR\ \nu_{max}^{nujol} cm^{-1}: 1730,\ 1670$$

MS (m/z): 167 ($Ph_2CH^+$)

(2) A solution of this product (10 2 g) in a mixture of tetrahydorfuran (24 ml) and water (0.6 ml) is cooled to -15 ° C and thereto is added sodium borohydride (1.0 g) and the mixture is stirred at -15 to -10 ° C for 20 minutes. To the mixture is added a mixture of ice-water (50 ml) and ethyl acetate (50 ml), and the pH value thereof is adjusted to pH 4 with aqueous oxalic acid solution. The ethyl acetate layer is collected and the aqueous layer is extracted with ethyl acetate. The ethyl acetate solutions are combined and washed with aqueous sodium hydrogen carbonate solution and water, and dried. The solvent is distilled off under reduced pressure to give 2-(2-furyl)-2-hydroxyacetic acid diphenylmethyl ester (10 g), as crystal.
Yield: 97 %
M.p.: 87 - 88 ° C

$$IR\ \nu_{max}^{nujol} cm^{-1}: 3450,\ 3410,\ 1740,\ 1600$$

MS (m/z): 308 ($M^+$)

(3)-(a) Phosphorus tribromide (1.01 g) is added dropwise to a suspension of this product (0.77 g) and potassium carbonate (0.5 g) in tetrahydrofuran (5 ml) under ice cooling and the mixture is stirred at the same temperature for 40 minutes. The mixture is filtered and to the filtrate are added 2N aqueous sodium hydroxide solution (7 ml) and ethyl acetate (7 ml), and the mixture is basified by adding aqueous sodium hydrogen carbonate thereto. The ethyl acetate layer is collected and the aqueous layer is extracted with ethyl acetate. The ethyl acetate solutions are combined and washed with saline solution. After drying, the solvent is distilled off under reduced pressure to give 2-(2-furyl)-2-bromoacetic acid diphenylmethyl ester (0.85 g, yield: 92 %) as a crude oily product. A solution of the crude product (0.85 g) in dimethylformamide (2 ml) is added dropwise to a suspension of N-hydroxyphthalimide potassium salt (0.60 g) in dimethylformamide (8 ml) at 0 ° C under argon atmosphere. Hereinafter the mixture is treated in the same manners as in Reference Example 1 - (1) and the residue is purified by silica gel column chromatography (solvent: hexane/ethyl acetate) to give 2-(2-furyl)-2-(phthalimidoxy)acetic acid diphenylmethyl ester (0.58 g) as

colorless crystal.
M.p.: 142 - 144 °C

$$IR\ \nu_{max}^{nujol} cm^{-1}: 1790, 1760, 1740$$

MS (m/z): 167 ($Ph_2CH^+$)

(3)-(b) A suspension of 2-(2-furyl)-2-hydroxyacetic acid diphenylmethyl ester (3.08 g), N-hydroxyphthalimide (2.69 g) and triphenylphosphine (3.93 g) in tetrahydrofuran (50 ml) is cooled to -55 °C and to this suspension is added dropwise azodicarboxylic acid diethyl ester (2.61 g) and then the mixture is stirred at -55 °C to 0 °C for 3.5 hours. The mixture is filtered and the filtrate is concentrated under reduced pressure to distill the solvent off. The residue is purified by silica gel chromatography (solvent: hexane/ethyl acetate) to give 2-(2- furyl)-2-(phthalimidoxy)acetic acid diphenylmethyl ester (2.87 g, yield: 63 %) as colorless crystal. The physicochemical properties of this product is identified with those of the product obtained in the above (a) procedure.

(4) A solution of this product (0.45 g) in methylene chloride (2ml) is cooled to -50 °C under argon atmosphere and thereto is added methylhydrazine (53 µl). The mixture is treated in the same manners as in Reference Example 1 - (2) to give 2-(2-furyl)-2-aminoxyacetic acid diphenylmethyl ester (0.30 g, yield: 92 %) as a light yellow oily product.

$$IR\ \nu_{max}^{nujol} cm^{-1}: 3320, 1750, 1600, 1580$$

MS (m/z): 324 ($MH^+$)

(5) This product (1.46 g) and 2-tritylaminothiazol-4-yl-glyoxylic acid (1.74 g) are dissolved in a mixture of acetonitrile (6 ml), tetrahydrofuran (16 ml) and water (6 ml) and treated in the same manners as in Reference Example 1 - (3) to give 2-(2-tritylaminothiazol-4-yl)-2-[(2-furyl)(diphenylmethyloxycarbonyl)-methyloxyimino]acetic acid (2.46 g), as crystal.
Yield: 81 %
M.p.: 142 - 143 °C (decomposed)

$$IR\ \nu_{max}^{nujol} cm^{-1}: 3230, 2800 - 2200, 1740, 1700, 1650, 1600$$

MS (m/z): 720 ($MH^+$)

Reference Example 3

(1) A mixture of thiophen-3-acetic acid (56.9 g), selenium dioxide (66.6 g), dioxane (500 ml), acetic acid (35 ml) and water (10 ml) is refluxed with stirring for 24 hours and filtered. The filtrate is concentrated and to the residue is added ethyl acetate (700 ml). The insoluble materials are filtered off and the filtrate is dried and then the solvent is distilled off. The residue is dissolved in tetrahydrofuran (1 ℓ) and thereto is added diphenyldiazomethane (103 g) under ice cooling over a period of 30 minutes. The mixture is stirred at room temperature for 1 hour and then filtered. The filtrate is concentrated and purified by silica gel column chromatography (solvent: hexane/ethyl acetate) to give (3-thienyl)glyoxylic acid diphenylmethyl ester (88.1 g).
Yield: 68 %
M.p.: 63 - 64 °C

$$IR\ \nu_{max}^{nujol} cm^{-1}: 1730, 1675$$

(2) A solution of this product (20 g) in a mixture of tetrahydrofuran (120 ml) and water (5 ml) is cooled

to -25° C and thereto is added sodium borohydride (2.35 g). The mixture is stirred at -25 to -5° C for 30 minutes and treated in the same manners as in Reference Example 2-(2) to give 2-(3-thienyl)-2-hydroxyacetic acid diphenylmethyl ester (19.5 g).
Yield: 97 %
M.p.: 93 - 96° C

$$IR\ \nu_{max}^{nujol} cm^{-1}: 3430, 1730$$

(3) A solution of this product (16.3 g), N-hydroxyphthalimide (13.5 g), triphenylphosphine (19.7 g) and azodicarboxylic acid diethyl ester (13.1 g) in tetrahydrofuran (250 ml) is treated in the same manners as in Reference Example 2-(3)-(b) to give 2-(3-thienyl)-2-(phthalimidoxy)acetic acid diphenylmethyl ester (17.8 g).
Yield: 76 %
M.p.: 120 - 121.5° C

$$IR\ \nu_{max}^{nujol} cm^{-1}: 1790, 1725$$

(4) A solution of this product (3.83 g) in methylene chloride (25 ml) and methylhydrazine (0.46 ml) are treated in the same manners as in Reference Example 1-(2) to give 2-aminoxy-2-(3-thienyl)acetic acid diphenylmethyl ester (2.77 g) quantitatively.

$$IR\ \nu_{max}^{nujol} cm^{-1}: 3315, 3260, 1750, 1590$$

(5) This product (13.0 g) and 2-tritylaminothiazol-4-yl-glyoxylic acid (14.5 g) are treated in the same manners as in Reference Example 1-(3) to give 2-(2-tritylaminothiazol-4-yl)-2-[(3-thienyl)(diphenylmethylcarbonyl)methyloxyimino]acetic acid (22.1 g).
Yield: 86 %,
M.p.: 149 - 151° C

$$IR\ \nu_{max}^{nujol} cm^{-1}: 3230, 1745, 1700$$

MS (m/z): 736 ($MH^+$)

Reference Example 4

(1) Sodium hydride (63 % oily dispersion, 7.7 g) is added to dimethylsulfoxide (60 ml) under argon atmosphere and the mixture is heated at 70 - 75° C for 45 minutes and then cooled. Tetrahydrofuran (50 ml) is added thereto and further is added dropwise a solution of furan-3-carboxylic acid methyl ester (10.6 g) in tetrahydrofuran (40 ml). The reaction mixture is stirred at a temperature from 2° C to room temperature for 1.5 hour and then acetic acid (15 ml) is added thereto under cooling. The solvent is distilled off under reduced pressure. To the residue are added acetic anhydride (160 ml) and sodium acetate (15 g) and the mixture is refluxed for 30 minutes. The solvent is distilled off and to the residue is added ethyl acetate. The insoluble materials are filtered off and the filtrate and the washings are concentrated to distill the solvent off. The residue (oily) is dissolved in methanol (200 ml) and thereto is added a suspension of lithium hydroxide (6 g) in water (20 ml). The mixture is stirred at room temperature for 1 hour. The mixture is adjusted to pH 1 - 2 with conc. hydrochloric acid under ice cooling and then the solvent is distilled off under reduced pressure. The residue is suspended in ether (200 ml) and methanol (50 ml) and thereto is added diphenyldiazomethane (14 g) over a period of 1 hour and the mixture is stirred at room temperature for 2 hours. The reaction solution is concentrated and thereto are added ethyl acetate and water, and the ethyl acetate layer is separated. The aqueous layer is extracted with ethyl acetate and the organic layers are

combined, washed with water, dried, and the solvent is distilled off. The residue is purified by silica gel column chromatography (solvent: hexane/ethyl acetate) to give 2-(3-furyl)-2-hydroxyacetic acid diphenylmethyl ester (3.65 g).
M.p.: 94 - 96° C

$$IR\ \nu_{max}^{nujol} cm^{-1}: 3450, 3400, 1740$$

(2) A solution of this product (4.2 g), N-hydroxyphthalimide (3.67 g), triphenylphosphine (5.36 g) and azodicarboxylic acid diethyl ester (3.56 g) in tetrahydrofuran (70 ml) is treated in the same manners as in Reference Example 2-(3)-(b) to give 2-(3-furyl)-2-(phthalimidoxy)acetic acid diphenylmethyl ester (4.82 g).
Yield: 78 %
M.p.: 116 - 117.5° C

$$IR\ \nu_{max}^{nujol} cm^{-1}: 1785, 1750, 1720$$

(3) A solution of this product (4.75 g) in methylene chloride (30 ml) and methylhydrazine (0.56 ml) are treated in the same manners as in Reference Example 1-(2) to give 2-aminoxy-2-(3-furyl)acetic acid diphenylmethyl ester (3.69 g) quantitatively.
Yield: 99 %

$$IR\ \nu_{max}^{nujol} cm^{-1}: 3330, 3260, 3150, 3065, 3030, 1750$$

(4) This product (3.32 g) and 2-tritylamino thiazol-4-yl-glyoxylic acid (14.5 g) are treated in the same manners as in Reference Example 1-(3) to give 2-(2-tritylaminothiazol-4-yl)-2-[(3-furyl)-(diphenylmethyloxycarbonyl)methyloxyimino]acetic acid (5.15 g).
Yield: 77 %
M.p.: 161 - 162° C

$$IR\ \nu_{max}^{nujol} cm^{-1}: 3240, 3110, 3060, 3030, 1750, 1700$$

MS (m/z): 720 ($MH^+$)

Reference Example 5

(1) Chelidonic acid (18.72 g), 4-aminoveratrol (15.58 g) and dimethylsulfoxide (100 ml) are heated to 160 -170° C and the solvent is distilled off. Chloroform is added to the residue and the mixture is extracted with diluted hydrochloric acid. The extract is basified and then extracted again with chloroform. The extract is washed, dried and the solvent is distilled off. To the residue is added a mixture of chloroform and ethyl acetate and the resulting crystals are collected to give 1-(3,4-dimethoxyphenyl)-4-pyridone (16.38 g).
M.p.: 168 - 170° C

(2) The product (8.0 g) obtained in the above procedure (1) is added to methylene chloride (100 ml) and the mixture is cooled. Thereto is added boron tribromide (17.38 g) dropwise and the mixture is stirred at the same temperature and then at room temperature. After stirring, the mixture is cooled again and methanol is added to the residue. The solvent is distilled off and the residue is recrystallized from methanol/ethyl acetate to give 1-(3,4-dihydroxyphenyl)-4-hydroxypyridinio bromide (9.40 g).
M.p.: 260 - 265° C (decomposed)

(3) The product (9.40 g) obtained in the above procedure (2), acetic anhydride (10.1 g) and pyridine (100 ml) are stirred at room temperature and the solvent is distilled off. The residue is dissolved in chloroform and washed with water. After drying, the solvent is distilled off. Ethyl acetate/hexane is added to

the residue and the precipitated crystals are collected by filtration to give 1-(3,4-diacetoxyphenyl)-4-pyridone (7.06 g).
M.p.: 162 - 165 °C

(4) The product (7.0 g) obtained in the above procedure (3), dimethoxyethane (120 ml) and pyridine (30 ml) are heated and thereto is added Lawesson's reagent, [2-4-bis(4-methoxyphenyl)-1,3-dithia-2,4-diphosph etane-2,4-disulfide] (5.92 g) and refluxed. The solvent is distilled off from the reaction solution and the residue is purified by silica gel chromatography. The thus obtained product is washed with ethyl acetate to give 1-(3,4-diacetoxyphenyl)-4-thiopyridone (4.76 g).
M.p.: 193 - 196 °C

(5) The product (0.50 g) obtained in the above procedure (4), water (4 ml), methanol (6 ml) and sodium hydrogen carbonate (0.35 g) are stirred under argon atmosphere. Hydrochloric acid is added to the reaction solution and the solvent is distilled off therefrom. The residue is purified by silica gel chromatography. The resulting product is washed with ethyl acetate to give 1-(3,4-dihydroxyphenyl)-4-thiopyridone (0.30 g).
M.p.: 259 - 261 °C (decomposed)

The corresponding starting compounds are treated in the same manners as in the above procedures (1) - (5) to give the following compounds.

(i) 1-(3,4-Dihydroxyphenethyl)-4-thiopyridone M.p.: 220 - 223 °C

(ii) 1-(4-Hydroxyphenyl)-4-thiopyridone

$$IR\ \nu_{max}^{nujol}\ cm^{-1}:\ 1620,\ 1120$$

Reference Example 6

4-Chloropyridine hydrochloride (7.5 g) is dissolved in water and the mixture is basified with aqueous sodium hydrogen carbonate solution, and then extracted with methylene chloride. The extract is dried and the solvent is distilled off under reduced pressure. Benzyl bromide (10.3 g) is added to the residue and stirred at room temperature for 2 hours. Ether is added to the mixture and the precipitates are filtered. The precipitates are dissolved in water and thereto is added 18 % sodium mercaptan (8 ml). The precipitates are filtered and purified by silica gel column chromatography to give 1-benzyl-4-thiopyridone (3.54 g).
M.p.: 184 - 185 °C (decomposed)

Reference Example 7

(1) To a solution of (R)-2-furyl-2-hydroxyacetic acid (571 mg, prepared from brucine salt) in tetrahydrofuran (10 ml) is added dropwise a solution of diphenyldiazomethane (820 mg) in tetrahydrofuran (5 ml) under ice cooling. The mixture is stirred at room temperature for 15 hours and then the solvent is distilled off under reduced pressure. The residue is purified by silica gel chromatography and further recrystallized from a mixture solvent of isopropyl ether/n-hexane to give (R)-2-furyl-2-hydroxyacetic acid diphenylmethyl ester (950 mg).
M.p.: 99 - 101 °C, $[\alpha]_D^{20}$ - 23.0°(C = 1.0, chloroform)

This product is treated in the same manners as the corresponding (R, S) isomer described in Reference Example 2-(3)-(b), (4) and (5) to give the following compounds.

(2) (S)-2-(2-Furyl)-2-(phthalimidoxy)acetic acid diphenylmethyl ester
M.p.: 114 - 115 °C,$[\alpha]_D^{20}$ + 88.9° (C = 1.0, chloroform)

(3) (S)-2-(2-Furyl)-2-aminoxyacetic acid diphenyl methyl ester
M.p.: 76 - 77 °C

(4) 2-(2-Tritylaminothiazol-4-yl)-2-[(S)-(2-furyl) (diphenylmethylcarbonyl)methyloxyimino] acetic acid
M.p.: 155 - 156 °C (decomposed)
$[\alpha]_D^{20}$ + 21.6°(C = 0.97, chloroform)

Reference Example 8

(1) To a solution of 2-furyl-2-hydroxyacetic acid diphenylmethyl ester (3.06 g) and pyridine (2.35 g) in

tetrahydrofuran (50 ml) is added dropwise a solution of (S)-1-(2-naphthylsulfonyl)pyrrolidine-2-carboxylic acid chloride (4.82 g) in tetrahydrofuran (15 ml) under ice cooling and the mixture is stirred for 1 hour. The solvent is distilled off under reduced pressure. Saline solution is added to the residue under ice cooling and the mixture is extracted with ethyl acetate, washed and then dried. The solvent is distilled off under reduced pressure and purified by silica gel chromatography, and then crystallized from ether and further recrystallized from a mixed solvent of ethyl acetate and isopropyl ether to give 2-(2S)-(2-furyl)-2-{(2S)-1-(2-naphthylsulfonyl)pyrrolidine-2-carbonyloxy}acetic acid diphenylmethyl ester (2.40 g) as colorless crystal.
M.p.: 145 - 146 °C

(2) This product (238 mg) is dissolved in a mixed solvent of tetrahydrofuran (5 ml) and methanol (5 ml) and the mixture is subjected to catalytic reduction in the presence of palladium-black (20 mg) at normal temperature under atmospheric pressure. The catalyst is filtered off and the solvent is distilled off under reduced pressure. The residue is powdered with n-hexane to give 2-(2S)-(2-furyl)-2-{(2S)-1-(2-naphthylsulfonyl)pyrrolidine-2-carbonyloxy}acetic acid (209 mg).

$$IR\ \nu_{max}^{nujol} cm^{-1}: 3500 - 3450, 3250 - 3200, 2700 - 2300, 1760 - 1730, 1330, 1150, 750$$

(3) To a solution of this product (1.54 g) in tetrahydrofuran (24 ml) is added 5 % aqueous sodium hydroxide solution (7.5 ml) under ice cooling and stirred for 2.5 hours. After concentration under reduced pressure, water is added to the mixture and the mixture is washed with ether. The aqueous layer is concentrated under reduced pressure and treated with strong acid ion-exchange resin (40 ml). The eluate is concentrated under reduced pressure and then cooled with ice. The precipitates are removed by filtration and the filtrate is distilled off under reduced pressure to give (S)-2-furyl-2-hydroxyacetic acid (484 mg).

(4) This product (484 mg) is esterificated in the same manners as in Reference Example 7-(1) to give (S)-2-furyl-2-hydroxyacetic acid diphenylmethyl ester (829 mg), as colorless needle.
Yield: 86 %
M.p.: 101 - 103 °C

This product is treated in the same manners as the corresponding (R, S)-isomer described in Reference Example 2-(3)-(b), (4) and (5) to give the following compounds.

(5) (R)-2-(2-Furyl)-2-(phthalimidoxy)acetic acid diphenylmethyl ester
M.p.: 115 - 116 °C

(6) (R)-2-(2-Furyl)-2-aminoxyacetic acid diphenyl methyl ester
M.P.: 68- 70 °C.

(7) 2-(2-Tritylaminothiazol-4-yl)-2-[(R)-(2-furyl) (diphenylmethyloxycarbonyl)methyloxy imino]acetic acid
M.p.: 145 - 146 °C (decomposed)

## Reference Example 9

To a suspension of 7-aminocephalosporanic acid (2.72 g) and quinoline (5.81 g) in methylene chloride (55 ml) is added dropwise trimethylsilyltrifluoromethane sulfonate (8.7 ml) under argon atmosphere. The mixture is stirred at room temperature for 4 hours and then the solvent is distilled off. Water (10 ml) is added to the residue and the mixture is subjected to HP-20 column chromatography (solvent: water). The fractions containing the desired product are collected and adjusted to pH 6.0 - 6.5 by adding aqueous sodium hydrogen carbonate soltuion. The mixture is concentrated and then purified by HP-20 column chromatrography (solvent: water/20 to 30 % aqueous methanol). The fractions containing the desired product are collected and concentrated. The residue is lyophilized to give 7-amino-3-(1-quinolinio)methyl-3-cephem-4-carboxylate (1.54 g) as light yellow powder.
M.p.: ~ 140 °C (decomposed)

$$IR\ \nu_{max}^{nujol} cm^{-1}: 3300 (br), 1760, 1610, 1590$$

## Claims

1. A cephalosporin compound of the formula:

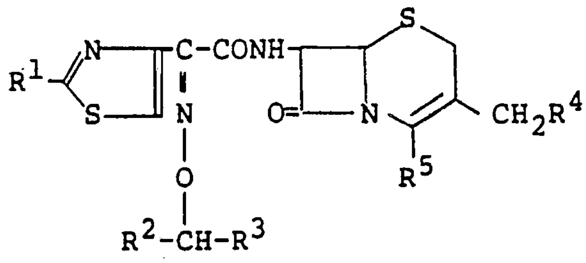

(I)

wherein $R^1$ is an amino group or protected amino group; $R^2$ is a substituted or unsubstituted heterocyclic group having 1 -3 hetero atoms selected from an oxygen atom and a sulphur atom; $R^3$ is a carboxyl group or a protected carboxyl group; $R^4$ is a nucleophilic compound residue; $R^5$ is a carboxyl group, a protected carboxyl group or a group of the formula: $-COO^-$, or a pharmaceutically acceptable salt thereof.

2. A compound as claimed in claim 1 wherein a nucleophilic residue for $R^4$ is an acetoxy group, a pyridinio group having optionally a substituent, a quinolinio group having optionally a substituent, a isoquinolinio group, 2,3-cyclopentapyridinio group, 5,6,7,8-tetrahydroquinolinio group, a phenantridinio group, a substituted thiazolinio group, a substituted tetrazolylthio group or a group of the formula:

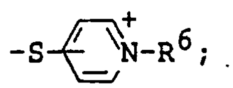

$R^6$ is a lower alkenyl group, a lower alkyl group having optionally a substituent, a phenyl group having optionally a substituent or nitrogen-containing heterocyclic group.

3. A compound as claimed in claim 2 wherein a nucleophilic residue for $R^4$ is an acetoxy group; a pyridinio group; a pyridinio group having 1 - 3 substituents selected from the group consisting of an amino group, a lower alkyl group, a lower alkoxy group, a carbamoyl group and a phenyl group; a quinolinio group; a quinolinio group having a substituent selected from a lower alkyl group, a lower alkoxy group and a halogen atom; an isoquinolinio group; 2,3-cyclopentanopyridinio group; 5,6,7,8-tetrahydroquinolinio group; a phenantridinio group; a lower alkyl substituted thiazolinio group; a lower alkyl substituted tetrazolylthio group or a group of the formula:

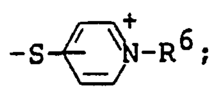

$R^6$ is a lower alkenyl group; a lower alkyl group; a lower alkyl group having a substituent selected from a lower alkylthio group, a phenyl group, a phenyl group substituted by hydroxy group, a thiocarbamoyl group and N-lower alkylthiocarbamoyl group; a phenyl group having a substituent selected from a hydroxy group and an amino group; or 2-oxo-pyrrolidinyl group.

4. A compound as claimed in claim 3 wherein $R^4$ is an acetoxy group, a pyridinio group, 3-amino-2-methyl pyridinio group, 5-amino-3-methoxypyridinio group, 3-carbamoylpyridinio group, 4-phenylpyridinio group, a quinolinio group, 6-chloroquinolinio group, 6-fluoroquinilinio group, 6-bromoquinolinio group, 6-methylquinolinio group, 6-methoxyquinolinio group, 3-bromoquinolinio group, 4-chloroquinolinio group, 4-methylquinolinio group, isoquinolinio group, 2,3-cyclopentenopyridinio group, 5,6,7,8-tetrahydroxyquinolinio group, phenantridinio group, 1-methyltetrazol-5-ylthio group, 4-methylthiazolinio group or a group of the formula:

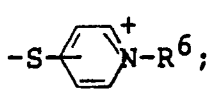

$R^6$ is a propenyl group, a methyl group, a methylthiomethyl group, a thiocarbamoylmethyl group, a

thiocarbamoylpropyl group, N-methylthiocarbamoylmethyl group, 4-hydroxyphenyl group, 3,4-dihydroxyphenyl group, 3-amino-4-hydroxyphenyl group, a benzyl group, 3,4-dihydroxyphenethyl group or 2-oxopyrrolidinyl group.

5. A compound as claimed in claim 4, wherein $R^1$ is an amino group; $R^3$ is a carboxyl group; $R^5$ is a carboxyl group or a group of the formula: $-COO^-$.

6. A compound as claimed in claim 5 wherein $R^2$ is a furyl group, a tienyl group or an aminothiazolyl group.

7. A compound as claimed in claim 6 wherein $R^2$ is a furyl group or a thienyl group.

8. A compound as claimed in claim 7 wherein $R^4$ is a quinolinio group, 6-chloroquinolinio group or a group of the formula:

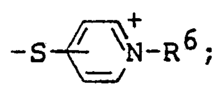

$R^6$ is a thiocarbamoylmethyl group, 3,4-dihydroxyphenyl group or 3,4-dihydroxyphenethyl group.

9. A compound as claimed in claim 8 which is 7β-{(Z)-2-(2-aminothiazol-4-yl)-2-[(2-furyl)(carboxy)-methyloxyimino]-acetamido}-3-[(1-quinolinio)methyl]-3-cephem-4-carboxylate.

10. A compound as claimed in claim 8 which is 7β-{(Z)-2-(2-aminothiazol-4-yl)-2-[((S)-2-furyl)(carboxy)-methyloxyimino]-acetamido}-3-[(1-quinolinio)methyl]-3-cephem-4-carboxylate.

11. A compound as claimed in claim 8 which is 7β-{(Z)-2-(2-aminothiazol-4-yl)-2-[(2-furyl)(carboxy)-methyloxyimino]-acetamido}-3-[(6-chloro-1-quinolinio)methyl]-3-cephem-4-carboxylate.

12. A compound as claimed in claim 8 which is 7β-{(Z)-2-(2-aminothiazol-4-yl)-2-[((S)-2-furyl)(carboxy)-methyloxyimino)-acetamido}-3-[(6-chloro-2-quinolinio)methyl)]-3-cephem-4-carboxylate.

13. A thiazolyl acetic acid derivative of the formula:

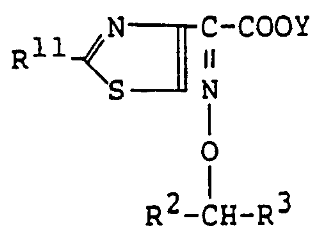

wherein $R^{11}$ is an amino group or a protected amino group; $R^2$ is a substituted or unsubstituted heterocyclic group having 1 - 3 hetero atoms selected from an oxygen atom and a sulfur atom; $R^3$ is carboxyl group or a protected carboxyl group; the group of the formula -COOY is a carboxyl group or a protected carboxyl group, or a salt thereof.

14. A process for preparing a cephalosporin compound of the formula:

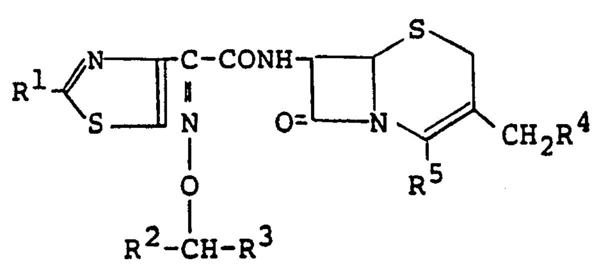

, (I)

wherein $R^1$ is an amino group or a protected amino group; $R^2$ is a substituted or unsubstituted heterocyclic group having 1 - 3 hetero atoms selected from the group consisting of an oxygen atom and a sulfur atom; $R^3$ is a carboxyl group or a protected carboxyl group; $R^4$ is a nucleophilic compound residue; $R^5$ is a carboxyl group, a protected carboxyl group or a group of the formula: $-COO^-$, or a pharmaceutically acceptable salt thereof, which comprises the step(s) of:

(A) condensing an oxyiminoacetic acid compound of the formula:

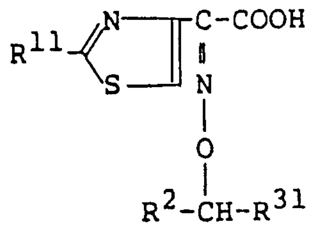

(II)

wherein $R^{11}$ is an amino group or a protected amino group; $R^{31}$ is a carboxyl group or a protected carboxyl group; $R^2$ is the same as defined above, a salt thereof or a reactive derivative thereof with a 7-aminocephalosporin compound of the formula:

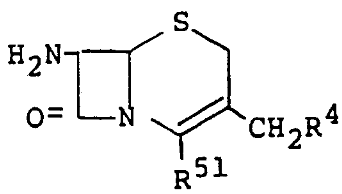

(III)

wherein $R^{51}$ is a carboxyl group, a protected carboxyl group or a group of the formula: $-COO^-$; $R^4$ is the same as defined above, or a salt, or

(B) reacting a cephalosporin compound of the formula:

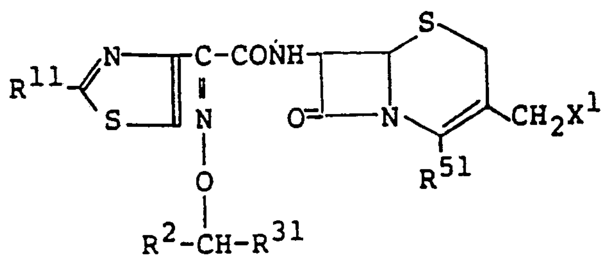

(IV)

wherein $X^1$ is a reactive group; $R^2$, $R^{11}$, $R^{31}$ and $R^{51}$ are the same as defined above, or a salt thereof with a nucleophilic compound (V) as herein defined or a salt thereof, or

(C) condensing a cephalosporin compound of the formula:

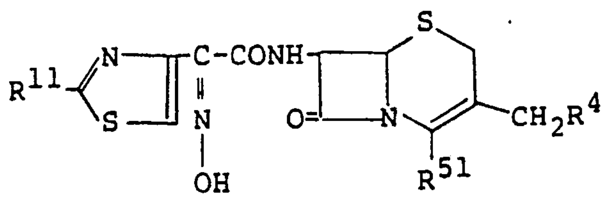

(VI)

wherein $R^{11}$, $R^4$ and $R^{51}$ are the same as defined above, or a salt thereof with a compound of the formula:

Z
|
$R^2$- CH-$R^{31}$ (VII)

wherein Z is a reactive group; $R^2$ and $R^{31}$ are the same as defined above, or a salt thereof, and

(D) when $R^{11}$ is a protected amino group and/or $R^{31}$ (and/or) $R^{51}$ are a protected carboxyl group, removing said protecting group therefrom, if desired, and

(E) converting the product into a pharmaceutically acceptable salt thereof, if desired.

15. A process for preparing a thiazolyl acetic acid derivative of the formula:

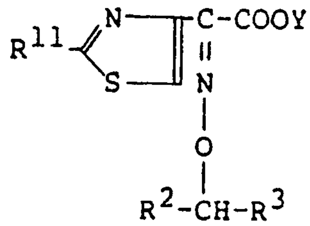

wherein $R^{11}$ is an amino group or a protected amino group; $R^2$ is a substituted or unsubstituted heterocyclic group having 1-3 hetero atoms selected from an oxygen atom and a sulfur atom; $R^3$ is a carboxyl group or a protected carboxyl group; the group of the formula: -COOY is a carboxyl group or a protected carboxyl group or a salt thereof which comprises

(A) reacting a compound of the formula:

Z
$R^2$- CH-$R^3$ (VII)

wherein Z is a reactive group with a compound of the formula:

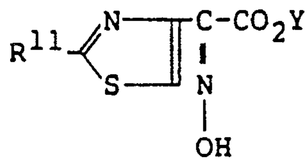

(VIII)

or (B) i) reacting a compound of the formula (VII) with an alkali metal salt of N-hydroxyphthalimide to give a compound of the formula:

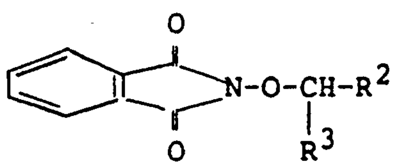

(X)

ii) treating the said compound (X) with a lower alkylhydrazine to give a compound of the formula:

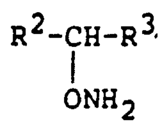

(XI)

iii) reacting the said compound (XI) with a glyoxylic acid compound of the formula:

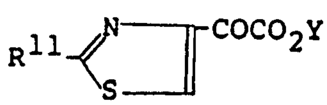

(XII)

and, if necessary, removing the protecting group from the said compound (XII).

16. A pharmaceutical composition which comprises an antimicrobially effective amount of the cephalosporin compound as defined in any of claims 1 to 12 or a salt thereof and a pharmaceutically acceptable carrier therefor.

17. Compounds of the general formula given in claim 1, or pharmaceutically acceptable salts thereof, for use in treating microbial infections in a warm-blooded animal.

18. A pharmaceutical composition comprising an antimicrobially effective amount of the cephalosporin compound as defined in any one of claims 1 to 12 or a salt thereof and a pharmaceutically acceptable

carrier therefor for use in treating microbial infections in a warm-blooded animal.